(19) Europäisches Patentamt
European Patent Office
Office européen des brevets

(11) Numéro de publication: **0 107 569**
**B1**

(12) **FASCICULE DE BREVET EUROPÉEN**

(45) Date de publication du fascicule du brevet: **18.03.87**

(51) Int. Cl.⁴: **C 07 D 487/06**, A 61 K 31/55 // C07D235/26 ,(C07D487/06, 235:00, 223:00)

(21) Numéro de dépôt: **83401976.2**

(22) Date de dépôt: **11.10.83**

(54) **Nouveaux dérivés de la 6-amino 7-hydroxy 4,5,6,7-tétrahydroimidazo/4,5,1-j-k/ /1/ benzazépin-2(1H)-one, leurs sels, leur préparation, leur application à titre de médicaments, les compositions les renfermant.**

(30) Priorité: **12.10.82 FR 8217054**

(43) Date de publication de la demande: **02.05.84 Bulletin 84/18**

(45) Mention de la délivrance du brevet: **18.03.87 Bulletin 87/12**

(84) Etats contractants désignés: **AT BE CH DE FR GB IT LI LU NL SE**

(56) Documents cité: **Néant**

(73) Titulaire: **ROUSSEL- UCLAF, 35, boulevard des Invalides, F-75007 Paris (FR)**

(72) Inventeur: **Frechet, Daniel, 45 rue Lecourbe, F-75015 Paris (FR)**
Inventeur: **Nedelec, Lucien, 45 boulevard de L'Ouest, F-93340 Le Raincy (FR)**
Inventeur: **Plassard, Guy, 6, rue Clément Marot, F-91600 Savigny- sur- Orge (FR)**
Inventeur: **Brown, Neil Leslie, 12, rue Jaucourt, F-75012 Paris (FR)**

(74) Mandataire: **Vieillefosse, Jean- Claude, Département des Brevets ROUSSEL UCLAF B.P no 9, F-93230 Romainville (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 107 569 B1

LIBER, STOCKHOLM 1987

**Description**

La présente invention concerne de nouveaux dérivés de la 6-amino 7-hydroxy 4,5,6,7-tétrahydroimidazo/4,5,1-j-k/ /1/ benzazépin-2(1H)-one, ainsi que leurs sels, leur préparation, l'application à titre de médicaments de ces nouveaux dérivés et les compositions les renfermant.

L'invention a pour objet de nouveaux dérivés de la 6-amino 7-hydroxy 4,5,6,7-tétrahydroimidazo/4,5,1-j-k/ /1/ benzazépin-2(1H)-one, ainsi que leurs sels d'addition avec les acides minéraux ou organiques, caractérisés en ce qu'ils répondent à la formule générale (I):

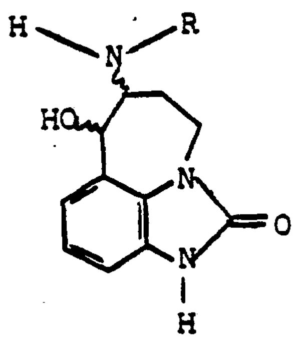

(I)

dans laquelle R représente un atome d'hydrogène, un radical alcoyle, linéaire ou ramifié, renfermant de 1 à 8 atomes dé carbone, éventuellement substitué par un radical hydroxy, par un radical phényle ou par un radical phényloxy ou un radical cycloalcoyle renfermant de 3 à 7 atomes de carbone, ou un radical 4-pipéridinyle dont l'atome d'azote porte éventuellement un substituant alcoyle renfermant de 1 à 4 atomes de carbone et les traits ondulés signifient que l'hydroxyle en position 7 et le radical amino en position 6 sont de configuration trans.

Dans la formule générale (I) et dans ce qui suit, le terme radical alcoyle, linéaire ou ramifié, renfermant de 1 à 8 atomes de carbone, désigne, par exemple, un radical n-pentyle, n-butyle, n-propyle, 2,2-diméthylpropyle ou de préférence, un radical isopropyle, éthyle ou méthyle;

le terme radical cycloalcoyle renfermant de 3 à 7 atomes de carbone désigne un radical cyclopropyle, cyclobutyle, cyclopentyle, cyclohexyle ou cycloheptyle;

Les sels d'addition avec les acides minéraux ou organiques peuvent être, par exemple, les sels formés avec les acides chlorhydrique, bromhydrique, nitrique, sulfurique, phosphorique acétique, formique, propionique, benzoïque, maléique, fumarique succinique, tartrique, citrique, oxalique, glyoxylique, aspartique, alcane sulfoniques tels que les acides méthane ou éthane sulfoniques, arylsulfoniques, tels que les acides benzène ou paratoluène sulfoniques et arylcarboxyliques.

Parmi les produits de l'invention, on peut citer ceux répondant à la formule générale (I), dans laquelle R représente un atome d'hydrogène ou un radical alcoyle renfermant de 1 à 5 atomes de carbone ainsi que leurs sels d'addition avec les acides minéraux ou organiques.

Parmi les produits objet de l'invention, on peut citer plus particulièrement:

- la (6RS, trans) 6-amino 7-hydroxy 4,5,6,7-tétrahydro imidazo/5,4,1-j-k/ /1/ benzazépin-2(1H)-one;
- la (6RS, trans) 6-éthylamino 7-hydroxy 4,5,6,7-tétrahydro imidazo/5,4,1-j-k/ /1/ benzazépin-2(1H)-one;
- la (6RS, trans) 6-méthylamino 7-hydroxy 4,5,6,7-tétrahydro-imidazo/5,4,1-j-k/ /1/ benzazépin-2(1H)-one;
- la (6RS, trans) 6-(1-méthyléthyl)amino 7-hydroxy 4,5,6,7-tétrahydro imidazo/5,4,1-j-k/ /1/ benzazépin-2-(1H)-one, ainsi que leurs sels d'addition avec les acides minéraux ou organiques.

L'invention a également pour objet un procédé de préparation des dérivés de formule générale (I), ainsi que de leurs sels, caractérisé en ce que l'on soumet le produit de formule (II):

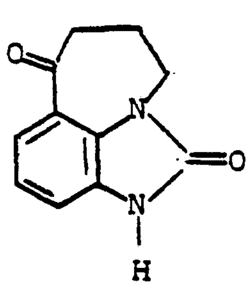

(II)

à l'action d'un nitrite d'alcoyle, de préférence en présence d'un acide, pour obtenir le produit de formule (III):

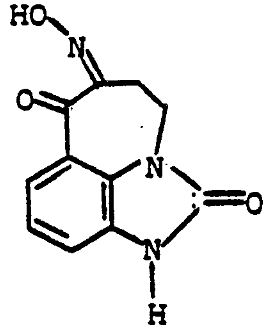

(III)

que l'on réduit, pour obtenir le produit de formule ($I_A$):

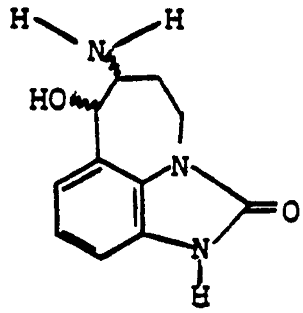

($I_A$)

dans laquelle les traits ondulés ont la signification déjà indiquée, que soit l'on isole, et si désiré, salifie, soit l'on soumet ou bien à une réaction d'alcoylation après blocage préalable du groupement $-NH_2$ de préférence sous forme de dérivé benzylé pour obtenir après déblocage un produit de formule ($I_B$):

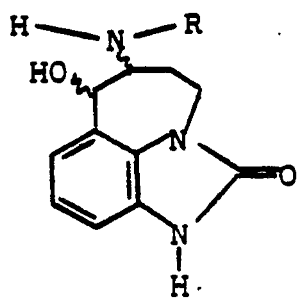

($I_B$)

dans laquelle les traits ondulés ont la signification déjà indiquée et R a la signification indiquée précédemment, à l'exception d'un atome d'hydrogène, que l'on isole et, si désiré, salifie ou bien à l'action d'un dérivé carbonylé de formule (IV):

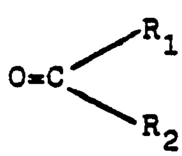

(IV)

dans laquelle $R_1$ et $R_2$ sont tels que 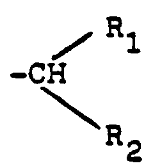

a la signification de R déjà indiquée, à l'exception d'un atome d'hydrogène et d'un radical méthyle en présence d'une amine tertiaire, puis à l'action d'un agent de réduction, pour obtenir un produit de formule ($I_C$):

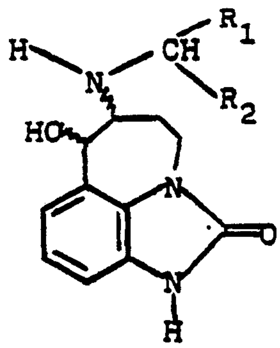

(I$_C$)

dans laquelle les traits ondulés ont la signification déjà indiquée et,

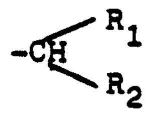

est défini comme ci-dessus, que l'on isole et, si désiré, salifie.

Dans des conditions préférentielles d'exécution du procédé ci-dessus, on opère comme suit:

- La réaction avec le nitrite d'alcoyle est effectuée de préférence à l'aide de nitrite de tert-butyle, mais on peut utiliser d'autres nitrites tel que celui d'isoamyle. La réaction peut être effectuée en présence d'une base ou de préférence en présence d'un acide, notamment l'acide chlorhydrique.
- La réduction du produit de formule (III) est de préférence effectuée par hydrogénation catalytique suivie ou non de l'action du borohydrure de sodium.
- La réaction d'alcoylation du produit de formule ($I_A$) peut avantageusement être effectuée après blocage, par exemple, sous forme de dérivé N-benzylé, du dérivé de formule ($I_A$), par action d'un halogénure d'alcoyle approprié, tel qu'un chlorure, un iodure, ou de préférence un bromure, en présence ou non d'un agent fixateur d'acides tel qu'un carbonate alcalin (par exemple le carbonate de sodium), un hydroxyde alcalin (par exemple, la potasse), ou une amine tertiaire (par exemple la triéthylamine), enfin élimination du groupement protecteur (par exemple, par hydrogénation catalytique dans le cas d'un radical benzyle).
- l'amine tertiaire que l'on fait réagir avec le dérivé carbonylé de formule (IV) est la triéthylamine.
- L'agent de réduction que l'on fait réagir sur le produit issu de l'action du produit de formule (IV) avec le produit de formule ($I_A$) est de préférence un borohydrure ou cyanoborohydrure alcalin, notamment de sodium.

L'invention a également pour objet un procédé de préparation des produits de formule générale (I), tel que défini précédemment, caractérisé en ce que pour préparer le produit de formule (II), l'on fait réagir le produit de formule (V):

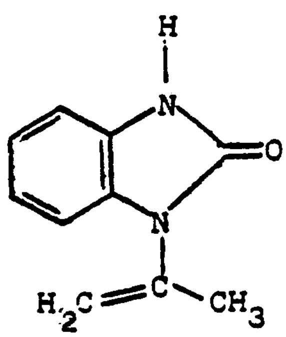

(V)

avec un 4-halogénobutyrate d'alcoyle de formule (VI):

$Hal\text{-}(CH_2)_3\text{-}COOalc$ (VI)

dans laquelle Hal représente un atome de chlore, de brome ou d'iode et alc un radical alcoyle renfermant de 1 à 4 atomes de carbone, puis hydrolyse en milieu acide le produit résultant de formule (VII):

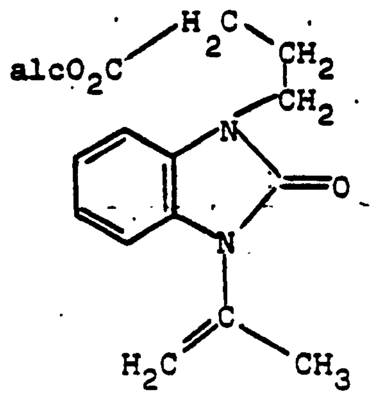

(VII)

dans laquelle alc est défini comme précédemment, pour obtenir le produit de formule (VIII):

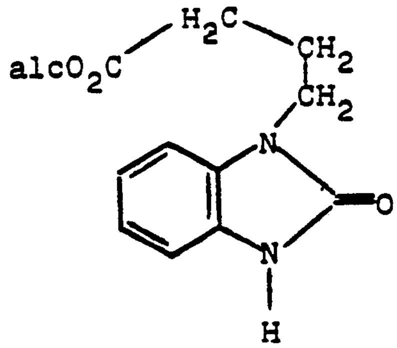

(VIII)

dont on saponifie la fonction ester pour obtenir l'acide de formule (IX):

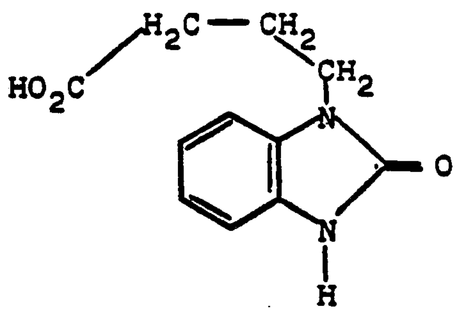

(IX)

dont on cyclise la chaîne latérale pour obtenir le produit de formule (II).

Dans des conditions préférentielles d'exécution du procédé ci-dessus:

- l'halogénobutyrate d'alcoyle est un bromobutyrate de méthyle ou d'éthyle et la condensation est effectuée en présence d'une base telle qu'un hydrure alcalin;
- l'hydrolyse du produit de formule (VII) est effectuée en présence d'un acide tel que l'acide sulfurique, la réaction étant effectuée au sein d'un alcool tel que l'éthanol;
- la saponification du produit de formule (VIII) est effectuée par une base telle que la soude ou la potasse au sein d'un alcool tel que le méthanol ou l'éthanol;
- la réaction de cyclisation de la chaîne latérale du produit de formule (IX) est effectuée soit en préparant un chlorure d'acide, par exemple, par action de chlorure de thionyle, que l'on traite par un acide de Lewis, par exemple, le chlorure d'aluminium, au sein d'un solvant organique tel que le chlorure de méthylène ou le dichloréthane, soit par action d'un agent déshydratant tel que l'acide polyphosphorique.

Le produit de départ de formule (V) est connu et décrit, par exemple dans J.Chem.Soc.Perkin I (1982) - 261.

Le dédoublement des dérivés racémiques de formule générale (I) peut être réalisé selon les procédés classiques.

Les dérivés de formule générale (I) présentent un caractère basique. On peut avantageusement préparer les sels d'addition des dérivés de formule générale (I), en faisant réagir en proportions sensiblement stoechiométriques, un acide minéral ou organique avec ledit dérivé de formule générale (I). Les sels peuvent être préparés sans isoler les bases correspondantes.

Les dérivés, objet de la présente invention, possèdent de très intéressantes propriétés pharmacologiques; ils sont doués notamment de remarquables propriétés antihypertensives et hypotensives et pour certains de

propriétés vasodilatatrices.

Ces propriétés sont illustrées plus loin dans la partie expérimentale.

Ces propriétés justifient l'utilisation des dérivés de la 6-amino 7-hydroxy 4,5,6,7-tétrahydro imidazo/4,5,1-j-k/ /1/ benzazépin-2(1H)-one, ainsi que leurs sels pharmaceutiquement acceptables, à titre de médicaments.

La présente invention a ainsi également pour objet l'application, à titre de médicaments, des dérivés de la 6-amino 7-hydroxy 4,5,6,7-tétrahydro imidazo/4,5,1-j-k/ /1/ benzazépin-2 (1H)-one, tels que définis par la formule générale (I) ainsi que de leurs sels d'addition avec les acides pharmaceutiquement acceptables.

Parmi les médicaments, objet de l'invention, on retient plus particulièrement les dérivés répondant à la formule générale (I), dans laquelle R représente un atome d'hydrogène ou un radical alcoyle, linéaire ou ramifié renfermant de 1 à 5 atomes de carbone, ainsi que leurs sels d'addition avec les acides pharmaceutiquement acceptables.

Parmi ces derniers, on retient tout particulièrement, les dérivés dont les noms suivent:
- la (6RS, trans) 6-amino 7-hydroxy 4,5,6,7-tétrahydroimidazo /5,4,1-j-k/ /1/ benzazépin-2(1H)-one;
- la (6RS, trans) 6-éthylamino 7-hydroxy 4,5,6,7-tétrahydro imidazo/5,4,1-j-k/ /1/ benzazépin-2(1H)-one;
- la (6RS, trans) 6-méthylamino 7-hydroxy 4,5,6,7-tétrahydro imidazo/5,4,1-j-k/ /1/ benzazépin-2(1H)-one;, - la (6RS, trans)6-(1-méthyléthyl)amino 7-hydroxy 4,5,6,7-tétrahydroimidazo/5,4,1-j-k/ /1/ benzazépin-2-(1H)-one, ainsi que leurs sels d'addition avec les acides pharmaceutiquement acceptables.

Les médicaments selon l'invention trouvent leur emploi, par exemple, dans le traitement de l'hypertension artérielle essentielle dans le traitement de l'hypertension de la cinquantaine, de la ménopause, du diabétique, de l'obèse et du pléthorique, ainsi que dans le traitement de l'hypertension artérielle du sujet âgé ou atteint d'artériosclérose et dans le traitement de l'hypertension d'origine rénale. Ils peuvent également être utilisés dans le traitement des insuffisances circulatoires périphériques, notamment dans le traitement de l'artérite des membres inférieurs, ainsi que dans le traitement de l'insuffisance circulatoire cérébrale, dans le traitement de la sénescence ou des artériopathies cérébrales.

La dose usuelle, variable selon le dérivé utilisé, le sujet traité et l'affection en cause peut être, par exemple, de 5 mg à 200 mg par jour, par voie orale chez l'homme du dérivé de l'exemple 2 pour le traitement de l'hypertension artérielle essentielle.

L'invention a enfin pour objet les compositions pharmaceutiques qui renferment au moins un dérivé précité ou l'un de ses sels d'addition avec les acides pharmaceutiquement acceptables, à titre de principe actif.

A titre de médicaments, les dérivés répondant à la formule générale (I) et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme, par exemple, les comprimés, simples ou dragéifiés, les gélules, les granulés, les suppositoires, les préparations injectables; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs. Les exemples qui suivent illustrent la présente invention, sans toutefois la limiter.

**Exemple 1: Chlorhydrate de (6RS, trans)6-amino 7-hydroxy 4,5, 6,7-tétrahydroimidazo/4,5,1-j-k/ /1/ benzazépin-2(1H)-one.**

**Stade A: 1,3 -dihydro 2-oxo 3-phénylméthyl 1H-benzimidazol-1-butanoate d'éthyle.**

On agite 29,5 g de 1,3-dihydro 1-phénylméthyl 2H-benzi-imidazol-2-one (décrit dans Helv. 2ctz <u>44</u> 1961 p.1278), dans 296 cm³ de diméthylformamide, ajoute en 30 minutes 7,6 g d'hydrure de sodium à 50 % dans l'huile, agite encore pendant 30 minutes, refroidit à +5°C, ajoute, goutte à goutte 33,9 g de 4-bromobutyrate d'éthyle en 15 minutes et agite encore pendant 3 heures à température ambiante. On verse la solution sur 500 cm³ d'eau glacée, extrait à l'éther, lave à l'eau, sèche, évapore, dissout l'huile ainsi obtenue dans 50 cm³ d'éther isopropylique, laisse cristalliser pendant 15 heures, essore et obtient 22,6 g du produit attendu (F ≈ 50°C). (F ≈ 52°C après recristallisation dans le cyclohexane).

**Analyse**: $C_{20}H_{22}N_2O_3$ = 338,39
Calculé: C % 70,98 H % 6,55 N % 8,28
Trouvé: 70,8 6,6 8,2.

**Stade B: Acide 1,3-dihydro 2-oxo 3-phénylméthyl 1H-benzimi-dazol -1-butanoïque.**

On porte au reflux pendant 3 heures sous atmosphère inerte 40,6 g de produit tel qu'obtenu ci-dessus, dans 400 cm³ de soude methanolique N, concentre à demi-volume, verse sur 1 litre d'eau glacée, acidifie à pH = 2 par addition d'acide chlorhydrique concentré, essore, lave, sèche et obtient 35,2 g du produit attendu F ≈ 166°C (168°C après recristallisation dans l'acétate d'éthyle.

**Analyse**: $C_{18}H_{18}N_2O_3$ = 310,33
Calculé: C % 69,66 H % 5,84 N % 9,02

Trouvé: 69,4 5,9 8,9.

**Stade C: 5,6-dihydro 1 = phénylméthyl imidazo/4,5-1-j-k/ /1/ benzazépin-2,7(1H,4H)-dione.**

On met en suspension 21,5 g du produit obtenu ci-dessus dans 450 $cm^3$ de chloroforme, ajoute 21,5 $cm^3$ de chlorure de thionyle, porte au reflux pendant une heure 15 minute et évapore à sec sous pression réduite.

On dissout le produit ainsi obtenu dans 860 $cm^3$ de dichloréthane sous atmosphère inerte, refroidit à +15°C, ajoute 18,67 g de chlorure d'aluminium, agite pendant 4 heures à 20°C, verse le mélange sur un litre d'eau glacée, additionnée de 43 $cm^3$ d'acide chlorhydrique concentré, agite pendant 10 minutes et filtre. On décante la phase organique, extrait la phase aqueuse au chlorure de méthylène, réunit les phases organiques, les lave par une solution aqueuse de carbonate de potassium à 10 % jusqu'à pH = 6, sèche, évapore à sec sous pression réduite, cristallise dans l'acétate d'éthyle et obtient après essorage 8,7 g du produit attendu (F= 135°C).

**Analyse**: (après recristallisation dans l'isopropanol) $C_{18}H_{16}N_2O_2$ = 292,32

Calculé: C % 73,95 H % 5,51 N % 9,58

Trouvé: 73,9 5,5 9,4.

**Stade D: 5,6-dihydroimidazo/4,5,1-j-k/ /1/ benzazépin-2,7 (1H,4H)-dione.**

On chauffe sous atmosphère inerte, à 150°C, 29,2 g du produit obtenu ci-dessus, 292 g d'acide o-phosphorique et 14,1 g de phénol, après 2 heures, refroidit le mélange à + 35°C environ, verse sous agitation sur 1200 $cm^3$ d'eau glacée, ajoute 2 litres de chlorure de méthylène, alcalinise à l'aide de lessive de soude, filtre et lave l'insoluble au chlorure de méthylène. On lave la phase organique, la sèche et l'évapore à sec sous pression réduite. On obtient 9,7 g du produit attendu (F = 235°C) après cristallisation puis chromatographie sous pression du filtrat (éluant: acétate d'éthyle - méthanol - triéthylamine 90-2-2).

**Analyse**: $C_{11}H_{10}N_2O_2$ = 202,20

Calculé: C% 65,33 H% 4,98 N% 13,85

Trouvé: 65,0 4,9 13,7

**Stade E: 6-oxime de 4,5-dihydroimidazo /4,5,1-j-k/ /1/ benzazépine-2, 6, 7(1H)-trione.**

On met en suspension, sous atmosphère inerte, 15,5 g du produit tel qu'obtenu ci-dessus, dans 620 $cm^3$ de tétrahydrofuranne, refroidit à +5°C, ajoute 42,5 $cm^3$ d'acide chlorhydrique éthanolique 1,8N et 10,5 $cm^3$ de nitrite de tert-butyle, agite 3h à +5°C, essore, lave au tétrahydrofuranne puis avec un mélange chloroforme-métanol (1-1) et obtient 16,5 g du produit attendu (F > 280°C).

**Stade F: chlorhydrate de (6RS-trans) 6-amino 7-hydroxy 4,5,6,7-tétrahydro imidazo /4,5,1-j-k/ /1/-benzazépin-2(1H)-one.**

On met en suspension 4g du produit obtenu ci-dessus et 2 g de palladium à 10 % sur charbon, dans 150 $cm^3$ de méthanol, agite sous hydrogène pendant 2 h 30mn, filtre, refroidit à l'aide d'un bain de glace, et ajoute lentement sous légère agitation 0,66 g de borohydrure de sodium, agite encore pendant 1 h 30 mn à +5°C et évapore à sec sous pression réduite à +30°C. On dissout le résidu obtenu ci-dessus dans 15 $cm^3$ de méthanol, acidifie à l'acétate d'éthyle chlorhydrique jusqu'à pH=1-2, essore et obtient 3,6 g du chlorhydrate attendu (F> 260°C). On recristallise dans le méthanol, puis dans l'éthanol (F> 260°C).

**Analyse**: $C_{11}H_{14}N_3C1O_2$ = 255,7

Calculé: C% 51,66 H% 5,51 N% 16,43 Cl% 13,86

Trouvé: 51,5 5,6 16,7 14,2.

**EXEMPLE 2: (6RS-trans) 6-éthylamino 7-hydroxy 4,5,6,7-tétra hydroimidazo /4,5,1-j-k/ /1/-benzazépin-2(1H)-one et son chlorhydrate.**

On met en suspension, sous agitation et atmosphère inerte, 5 g du produit de l'exemple 1, dans 200 $cm^3$ de méthanol, ajoute 2,18 $cm^3$ d'acétaldéhyde et 10 $cm^3$ de triéthylamine, agite pendant 1 heure à température ambiante, refroidit à l'aide d'un bain de glace, ajoute en 1 heure 10 g de borohydrure de sodium, et agite encore 30 mn à +5°C et 1 heure à température ambiante. On évapore le méthanol, reprend le résidu au chlorure de méthylène, lave à l'eau puis avec une solution aqueuse saturée de chlorure de sodium, sèche, évapore à sec sous pression réduite, christallise dans l'acétate d'éthyle, et obtient, après essorage 2,6 g -- du produit attendu (F ≈ 190°C).

**Formation du chlorhydrate**

On dissout 4,2 g de base telle qu'obtenue ci-dessus, dans 80 $cm^3$ d'éthanol au reflux, refroidit à l'aide d'un bain de glace, ajoute 10cm$^3$ d'acétate d'éthyle chlorhydrique, évapore à sec, reprend par 30 $cm^3$ d'éther, essore et recristallise dans l'éthanol. On obtient ainsi 3,14 g du produit attendu (F ≈ 230°C).

**Analyse**: $C_{13}H_{18}N_3O_2Cl$ = 283,75

Calculé: C% 55,02 H% 6,39 N% 14,81 Cl% 12,49

Trouvé: 55,1 6,4 14,4 12,3

**EXEMPLE 3: chlorhydrate de (6RS-trans) 7-hydroxy 6-méthylami no 4,5,6,7-tétrahydroimidazo /5,4,1-j-k/ /1/-benzazépin-2(1H)one.**

**Stade A: (6RS-trans) 7-hydroxy 6-(phénylméthyl)amino 4,5,6,7-tétrahydroimidazo /5,4,1-j-k/ /1/benzazépin-2(1H)-one.**

On agite à température ambiante pendant 1 h, sous atmosphère inerte, 1 g du produit de l'example 1 dans 13 $cm^3$ de méthanol, 2 $cm^3$ de triéthylamine et 1 $cm^3$ de benzaldéhyde. On glace, ajoute en 15 mn 1 g de borohydrure de sodium, agite encore pendant 45 mn, ajoute 100 $cm^3$ d'eau, extrait au chlorure de méthylène, lave à l'eau, sèche, filtre et évapore à sec sous pression réduite. On chromatographie sur silice l'huile obtenue ci-dessus (éluant: acétate d'éthyle-méthanol-triéthylamine 90-5-5), cristallise dans l'acétate d'éthyle, et obtient 820 mg du produit attendu (F ≈ 145° C).

**Analyse**: (après recristallisation dans l'acétate d'éthyle) $C_{18}H_{19}N_3O_2$ = 309,36

Calculé: C% 69,88 H% 6,19 N% 13,58

Trouvé: 69,6 6,2 13,4

**Stade B: (6RS-trans) 7-hydroxy 6-/méthyl (phénylméthyl)amino/ 4,5,6,7-tétrahydroimidazo /5,4,1-j-k/ /1/-benzazépin-2(1H)one.**

On dissout 4,6 g de produit tel qu'obtenu ci-dessus, dans 69 $cm^3$ de méthanol et 11,5 $cm^3$ de triéthylamine, fait barboter du bromure de méthyle pendant 2 heures à température ambiante et évapore à sec sous pression réduite. On reprend par 100 $cm^3$ de solution saturée de bicarbonate de sodium, extrait au chlorure de methylène, lave a l'eau, sèche, évapore a sec sous pression réduite, cristallise dans l'acétate d'éthyle, et obtient, après essorage, 4,3 g du produit attendu(F= 228° C) (F= 232° C après recristallisation dans l'éthanol).

**Analyse**: $C_{19}H_{21}N_3O_2$ = 323,38

Calculé: C% 70,56 H% 6,55 N% 13,00

Trouvé: 70,3 6,5 12,8

**Stade C: chlorhydrate de (6RS-trans) 7-hydroxy 6-méthylamino 4,5,6,7-tétrahydro imidazo /5,4,1-j-k/ /1/ benzazépin-2(1H)one.**

On dissout 450 mg du produit obtenu ci-dessus dans 45 $cm^3$ d'éthanol, ajoute 225 mg de palladium à 10 % sur charbon, agite sous hydrogène pendant 1 heure, filtre, et évapore à sec sous pression réduite. On dissout l'huile ainsi obtenue, dans 5 $cm^3$ d'éthanol, filtre, ajoute un excès d'acétate d'éthyle chlorhydrique, laisse cristalliser, essore, lave à l'éthanol, recristallise dans le méthanol, et obtient 110 mg du produit attendu (F= 280-290° C).

**Analyse**: $C_{12}H_{16}ClN_3O_2$ = 269,93

Calculé: C% 53,43 H% 5,98 Cl% 13,15 N% 15,58

Trouvé: 53,2 5,9 13,4 15,4

**Exemple 4: Chlorhydrate de (6RS,trans) 7-hydroxy 6-propylamino 4,5,6;7-tétrahydroimidazo/4,5,1,j-k/ /1/ benzazépin-2(1H)-one.**

On opère comme à l'exemple 2 en remplaçant l'acétaldéhyde par de l'aldéhyde propionique. On reprend 5,1 g de produit brut obtenu par 15 $cm^3$ d'éthanol, ajoute 15 $cm^3$ d'acétate d'éthyle chlorhydrique puis 30 $cm^3$ d'acétate d'éthyle. On essore 3,8 g de chlorhydrate attendu. F = ≈ 210° C.

**Exemple 5: Chlorhydrate de (6RS,trans) 7-hydroxy 6-butyl amino 4,5,6,7-tétrahydroimidazo/4,5,1-j-k/ /1/ benzazépin-2(1H)-one.**

On opère comme à l'exemple 2, en partant d'aldéhyde butylique. On reprend 2,5 g de base obtenue dans 10 $cm^3$ d'éthanol et ajoute 10 $cm^3$ d'acétate d'éthyle chlorhydrique. On essore 1,5 g de produit attendu. F = ≈ 210° C.

**Exemple 6: Chlorhydrate de (6RS,trans) 7-hydroxy 6-/(2,2 diméthylpropyl)amino/ 4,5,6,7-tétrahydroimidazo/4,5,1-j-k/ /1/ benzazépin-2(1H)-one.**

On opère comme à l'exemple 2, en partant de l'aldéhyde pivalique et prépare le chlorhydrate comme à l'exemple 5. On obtient 2,2 g de produit attendu. F = ≈ 260° C.

**Exemple 7: (6R8,trans) 7-hydroxy 6-/(2-hydroxyéthyl)amino/ 4,5,6,7-tétrahydroimidazo/4,5,l-j-k/ /l/ benzazépin-2(1H)-one et son chlorhydrate.**

On agite une heure à température ambiante, 10,24 g de chlorhydrate de (6RS,trans) 6-amino 7-hydroxy 4,5,6,7-tétrahydroimidazo/4,5,l-j-k/ /1/ benzazépin-2-(1H)-one obtenu à l'exemple 1, 4,8 g de glycolaldéhyde, 14 $cm^3$ de triéthylamine et 400 $cm^3$ de méthanol. On refroidit à 0°/5°C, ajoute en 15 minutes 10,24 g de borohydrure de sodium. On agite 45 minutes à température ambiante, ajoute 100 $cm^3$ d'eau, concentre sous pression réduite jusqu'à 100 $cm^3$ et laisse cristalliser deux heures à température ambiante. On essore et obtient 6,3 g de produit brut. F = 220°C.

On prépare le chlorhydrate comme aux exemples 4 et 5 à partir de 450 mg de base et obtient 280 mg de chlorhydrate, F = 242°C, que l'on recristallise dans le méthanol à reflux. On recueille 67 mg de chlorhydrate. F = 245°C.

**Exemple 8: Chlorhydrate de (6RS,trans) 7-hydroxy 6-cyclohexyl amino 4,5,6,7-tétrahydro imidazo/4,5,l-j-k/ /l/ benzazépin-2 (1H)-one.**

On opère comme à l'exemple 2 en partant de cyclohexanone.

On prépare le chlorhydrate comme à l'exemple 4 à partir de 6,2 g de la base obtenue et obtient 4,2 g de produit attendu, F ≈ 280°C, que l'on recristallise dans le méthanol. On recueille 3,4 g de produit. F = 280°C.

**Exemple 9: Chlorhydrate de (6RS,trans) 7-hydroxy 6-/(phényl méthyl)amino/4,5,6,7-tétrahydroimidazo/4,5,l-j-k/ /1/ benzazépin-2(lH)-one.**

On opère comme au stade A de l'exemple 3 et obtient la base attendue. F = 145°C. On en dissout 3,5 g dans 70 $cm^3$ d'isopropanol au reflux, glace, ajoute peu à peu un excès d'acétate d'éthyle chlorhydrique. On essore 3,85 g de chlorhydrate attendu. F ≈ 240°C après recristallisation dans l'isopropanol.

**Exemple 10: Chlorhydrate de (6RS,trans) 7-hydroxy 6-/(2phényléthyl)amino/4,5,6,7-tétrahydroimidazo/4,5,1-j-k/ /1/ benzazépin-2 (1H)-one.**

On opère comme à l'exemple 2 en partant de phényl acétaldéhyde, chromatographie sur silice la base obtenue en éluant par un mélange chlorure de méthylène - méthanol (9/1) et prépare le chlorhydrate comme à l'exemple 4 et obtient le chlorhydrate attendu. F = 190°C.

**Exemple 11: Chlorhydrate de (6RS,trans) 7-hydroxy 6-/(3phényl propyl)amino/4,5,6,7-tétrahydroimidazo/4,5,1-j-k/ /1/ benzazépin-2(1H)-one.**

On opère comme à l'exemple 2 à partir de l'aldéhyde 3-phényl propylique et obtient la base que l'on transforme en 2,1 g de chlorhydrate. F = 230°C après recristallisation dans l'éthanol.

**Exemple 12: Chlorhydrate de (6RS,trans) 7-hydroxy 6-/(2phenoxy éthyl)amino/4,5,6,7-tétrahydroimidazo/4,5,1-j-k/ /1/ benzazépin-2(1H)-one.**

On opère comme à l'exemple 2 à partir de phénoxy acétaldéhyde puis chromatographie sur silice la base obtenue en éluant par un mélange chlorure de méthylène -méthanol (9/1). On recristallise la fraction intéressante dans l'isopropanol et obtient la base, F ≅ 150°C. On prépare le chlorhydrate en dissolvant la base dans du chlorure de méthylène et de l'isopropanol à reflux, concentre et ajoute de l'acétate d'éthyle chlorhydrique. On laisse cristalliser deux heures à température ambiante et essore le chlorhydrate attendu. F ≈ 250°C.

**Exemple 13: Dichlorhydrate de (6RS,trans) 7-hydroxy 6-/(1 méthyl 4-pipéridinyl)amino/4,5,6,7-tétrahydroimidazo/4,5,1-jk/ /1/ benzazépin-2-(1H)-one.**

On agite 24 heures à température ambiante 7,6 g de (6RS, trans) 7-hydroxy 6-amino 4,5,6,7-tétrahydroimidazo/4,5,1-j-k/ /1/ benzazépin-2(1H)-one, 7,6 $cm^3$ N-méthyl 4-pipéridone, 380 $cm^3$ de méthanol et 7,6 g de cyanoborohydrure de sodium. On refroidit à température inférieure à 0°C, ajoute goutte à goutte, 5 $cm^3$ d'acide chlorhydrique concentré, agite dix minutes à température ambiante, refroidit à nouveau à 0°C et alcalinise à pH 8 avec de la lessive de soude. On évapore le méthanol, reprend le résidu par du chlorure de méthylène à 10 % de méthanol, filtre l'insoluble et évapore à sec. On chromatographie le résidu sur silice en éluant par un mélange d'acétate d'éthyle - méthanol - ammoniaque (80-15-5) et obtient 7,3 g de base attendue. On dissout la base dans 40 $cm^3$ d'éthanol ajoute 20 $cm^3$ d'éthanol chlorhydrique, essore et recueille 7 g de chlorhydrate. F ≈ 260°C.

**Analyse**: $C_{17}H_{24}N_4O_2$ 2HCl PM: 389,32
Calculé: C % 52,44 H % 6,73 N % 14,36 Cl % 18,21
Trouvé: 52,5 7 14,3 18,0.

**Exemple 14: Chlorhydrate de (6RS,trans) 7-hydroxy 6-/(1-méthyl éthyl)amino/4,5,6,7-tétrahydroimidazo/4,5,1-j-k/ /1/ benza zépin-2(1H)-one.**

On refroidit à 0/+5°C, 6 g de chlorhydrate de (6RS,trans) 7-hydroxy 6-amino 4,5,6,7-tétrahydroimidazo/4,5,1-j-k/ /1/ benzazépin-2(1H)-one dans 60 $cm^3$ de méthanol et 30 $cm^3$ d'acétone. En un quart d'heure, on ajoute à 0/+5°C, 3 g de cyanoborohydrure de sodium, puis agite trois heures à température ambiante. On évapore à sec sous pression réduite, ajoute 60 $cm^3$ d'eau et extrait au chloroforme. On sèche la phase organique, évapore à sec et obtient 3,6 g de produit (F ≈ 166°C) que l'on reprend par 60 $cm^3$ d'éthanol. On ajoute de l'acétate d'éthyle chlorhydrique, essore et obtient 3,7 g de chlorhydrate attendu. F ≈ 270°C. On recristallise dans l'acétate d'éthyle après dissolution à chaud dans le méthanol et on recueille 3,3 g du produit attendu. F ≈ 270°C.

**Analyse**: $C_{14}H_{20}N_3O_2Cl$ PM = 297,78
Calculé: C % 56,46 H % 6,77 N % 14,11 Cl % 11,90
Trouvé: 56,5 6,9 14,0 12,1.

**Exemple 15: 4,5-dihydroimidazo/4,5,1-j-k/ /1/ benzazépin-2,7(1H,6H)-dione.**

**Stade A: 2,3-dihydro 3-(1-méthyléthényl)2-oxo 1H-benzimidazol-1-butanoate-d'éthyle.**
On agite 5,75 g d'hydrure de sodium à 50 % dans l'huile dans 10 $cm^3$ de diméthylformamide, ajoute en 45 minutes en maintenant la température à 20°C ± 2 °C/19 g de 1,3-dihydro 1-(1-méthyl éthényl)2H-benzimidazol-2-one [décrit dans J.Chem.Soc.Perkin (1982), 261] dans 150 $cm^3$ de diméthylformamide et maintient l'agitation pendant encore une demi-heure. On ajoute en quinze minutes, 23,4 g de 4-bromobutyrate d'éthyle et agite quatre heures à température ambiante. On verse le mélange réactionnel sur 800 $cm^3$ d'eau glacée, extrait à l'éther, lave à l'eau, sèche et concentre à sec. On obtient 33 g de produit attendu.

**Stade B: 2,3-dihydro 2-oxo 1H-benzimidazol-1-butanoate d'éthyle.**
On mélange 15,5 $cm^3$ d'acide sulfurique dans 155 $cm^3$ d'éthanol, refroidit à 0/+5°C, ajoute 31,4 g du produit obtenu ci-dessus et agite 5 heures à 0/+5°C. On neutralise par de la lessive de soude, verse le mélange réactionnel sur 1,5 litre d'eau glacée et agite 5 minutes. On essore, lave à l'eau et obtient 22 g de produit attendu. F = 88°C.

**Stade C: Acide 2,3-dihydro 2-oxo 1H-benzimidiazol-1-butanoïque**
On dissout 22 g de produit obtenu au stade B dans 22 $cm^3$ de lessive de soude et 200 $cm^3$ de méthanol. On porte au reflux pendant une heure, évapore le méthanol et reprend le résidu par 1,5 litre d'eau glacée. On acidifie à pH 1 par de l'acide chlorhydrique concentré, essore le précipité formé, lave à l'eau et recueille 18,1 g de produit. F = 180°C. On recristallise dans de l'isopropanol et obtient 15,2 g de produit attendu. F = 185°C.

**Stade D: 4,5-dihydro imidazo/4,5,1-j-k/ /1/ benzazépin-2,7 (1H,6H)-dione.**
On porte au reflux 300 $cm^3$ de chloroforme, 15,2 $cm^3$ de chlorure de thionyle et 15,2 g de l'acide obtenu au stade précédent, pendant une heure et demie. On refroidit à +15°C et ajoute en une seule fois 18,4 g de chlorure d'aluminium. On agite 4 heures à température ambiante et verse le mélange réactionnel sur 600 $cm^3$ d'eau glacée contenant 15 $cm^3$ d'acide chlorhydrique concentré, agite 5 minutes et essore le précipité formé et obtient 9,9 g de produit attendu brut. On récupère 2,5 g des liqueurs-mères. L'ensemble des produits obtenu est recristallisé dans de l'isopropanol. On obtient 8,5 g de produit attendu. F = 238°C.

**Exemples de compositions pharmaceutiques.**

**Exemple A**:

On a préparé des comprimés répondant à la formule:

- Chlorhydrate de (6RS,trans) 6-méthylamino7-hydroxy 4,5, 6,7-tétrahydro imidazo/5,4,1-j-k/ /1/ benzazépin-2-(1H)-one
........................................................ 10 mg
- Excipient q.s. pour un comprimé terminé à...... 100 mg

(Détail de l'excipient: lactose, amidon, talc, stéarate de magnésium).

**Exemple B**:

On a préparé des comprimés répondant à la formule:

- Chlorhydrate de (6RS,trans) 6-éthylamino 7-hydroxy 4,5,6,7-tétrahydro imidazo/5,4,1-j-k/ /1/ benzazépin-2(1H)-one
........................................................ 20 mg
- Excipient q.s. pour un comprimé terminé à...... 100 mg

(Détail de l'excipient: lactose, amidon, talc, stéarate de magnéisum).

**Exemple C**:

On a préparé des comprimés répondant à la formule:

- Chlorhydrate de (6RS,trans)6-(1-méthyléthyl)amino 7-hydroxy 4,5,6,7-tétrahydroimidazo/5,4,1-j-k/ /1/ benzazépin-2(1H)-one
........................................................ 10 mg
- Excipient q.s. pour un comprimé terminé à...... 100 mg

(Détail de l'excipient: lactose, amidon, talc, stéarate de magnésium).

**Etude pharmaceutique**

**1) Détermination de l'activité hypotensive**

L'activité hypotensive a été étudiée sur des rats mâles de souche WISTAR pesant 300 g environ et anesthésiés au pentobarbital sodique (50 mg/kg par voie intraveineuse).

Le produit testé a été administré par voie intraveineuse dans la veine pénile ou la veine jugulaire.

La pression artérielle carotidienne a été mesurée avant et après administration du produit testé.

Le tableau ci-après indique les variations exprimées en pourcentage de la pression artérielle après administration du produit testé par rapport à la pression artérielle témoin initial.

| Produits de l'exemple | Dose mg/kg | Variations % de la pression artérielle | | | |
|---|---|---|---|---|---|
| | | 1 minute après l'adminis-tration | 5 minutes après l'adminis-tration | 10 minutes après l'adminis-tration | 30 minutes après l'adminis-tration |
| 1 | 10 | - 54 | - 46 | - 43 | - 30 |
| | 1,0 | - 25 | - 11 | - 11 | - 9 |
| 2 | 10 | - 59 | - 53 | - 54 | - 51 |
| | 1,0 | - 34 | - 23 | - 19 | - 18 |
| | 0,1 | - 18 | - 7 | - 9 | - 7 |
| 3 | 1,0 | - 33 | - 21 | - 22 | - 17 |
| 4 | 1,0 | - 42 | - 23 | - 9 | - 5 |
| 5 | 1,0 | - 36 | - 16 | - 9 | - 4 |
| 6 | 10 | - 44 | - 32 | - 32 | - 23 |
| | 1,0 | - 21 | - 7 | - 4 | + 5 |
| 7 | 10 | - 53 | - 29 | - 29 | - 19 |
| 10 | 1,0 | - 57 | - 41 | - 24 | - 19 |
| 11 | 1,0 | - 52 | - 48 | - 49 | - 48 |
| 12 | 1,0 | - 14 | - 16 | - 20 | - 20 |
| 14 | 1,0 | - 45 | - 34 | - 30 | - 22 |
| | 0,1 | - 26 | - 17 | - 4 | - 5 |

**2) Action vasodilatatrice périphérique**

**Méthode**

On pratique la mesure directe de la vasomotricité par étude de la variation de pression dans un circuit vasculaire isolé perfusé à débit constant suivant la technique suivante:

L'étude est réalisée sur des chiens Beagle des deux sexes pesant 10 à 16 kg anesthésiés au pentobarbital sodique à la dose de 35 mg/kg i.v. Une perfusion de nembutal, 4,5 mg/4,5 ml/kg par heure, est mise en place dans la veine saphène pendant la durée de l'expérience. Les chiens sont intubés avec une canule trachéale et ventilés. Après administration i.v. de 5000 UI d'héparine, l'artère fémorale droite est cathétérisée et le sang qui s'en écoule après passage au travers d'un circuit extra-corporel le plus court possible et réintroduction dans l'artère fémorale gauche, vient perfuser la patte postérieure gauche. Sur le circuit extra-corporel sont placés une pompe à débit constant en aval et en dérivation un capteur de pression permettant d'enregistrer la pression de perfusion dans la patte.

La pression artérielle est prise à la carotide à l'aide d'un autre capteur de pression.

Le débit de perfusion dans la patte est ajusté de manière à égaliser les pressions carotidienne et fémorale.

La patte postérieure gauche est placée dans un sac contenant de la glace pilée, ce qui entraîne une augmentation de la pression de perfusion. La sensibilité de la patte est testée psr l'administration de naftidrofuryl à 100, 300, 1000 et 3000 µg dans un volume de 0,2 ml d'eau distillée. Le produit est injecté dans la circulation extra-corporelle en amont de la pompe à diverses doses.

Après l'administration de la gamme de doses du produit, une nouvelle gamme de doses de naftidrofuryl est injectée permettant de vérifier la stabilité de la préparation.

Les diminutions de pression traduisent une diminution du tonus de la zone perfusée donc des résistances périphériques.

Les effets sont exprimés par les variations en pourcentage de la pression de perfusion de la patte postérieure.

Résultats:

Sur ce modèle d'étude, les produits des exemples 3 et 14 sont respectivement 40 à 50, et 300 fois plus actifs que le naftidiofuryl.

**3) Etude de la toxicité aigue**

On a évalué les doses létales $DL_0$ des différents composés testés après administration par voie orale chez la souris.

On appelle $DL_0$ la dose maximale ne provoquant aucune mortalité en 8 jours.

**Les résultats obtenus sont les suivants:**

| Produit de l'exemple | $DL_0$ en mg/kg |
|---|---|
| 1 | > 400 |
| 2 | > 400 |
| 3 | 200 |
| 4 | 200 |
| 5 | 20 |
| 6 | 60 |
| 7 | > 400 |
| 10 | 20 |
| 11 | 80 |
| 12 | 100 |
| 13 | > 400 |
| 14 | 200 |

**Revendications** pour les Etats contractants BE CH DE PR GB IT LI LU NL SE

1) Les dérivés de la 6-amino 7-hydroxy 4,5,6,7-tétrahydro-imidazo/4 5 1-j-k/ /1/ benzazépin-2-(1H)-one ainsi que leurs sels d'addition avec les acides minéraux ou organiques, caractérisés en ce qu'ils répondent à la formule générale (I):

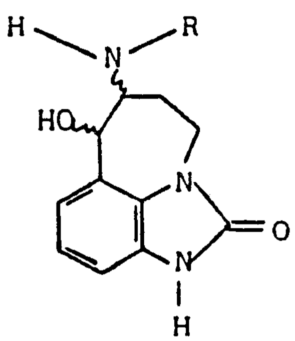

(I)

dans laquelle R représente un atome d'hydrogène, un radical alcoyle, linéaire ou ramifié renfermant de 1 à 8 atomes de carbone, éventuellement substitué par un radical hydroxy, par un radical phényle ou par un radical phényloxy ou un radical cycloalcoyle renfermant de 3 à 7 atomes de carbone, ou un radical 4-pipéridinyle dont l'atome d'azote porte éventuellement un substituant alcoyle renfermant de 1 à 4 atomes de carbone et les traits ondulés signifient que l'hydroxyle en position 7 et le radical amino en position 6 sont de configuration trans.

2) Les dérivés répondant à la formule générale (I) de la revendication 1, dans laquelle R représente un atome d'hydrogène ou un radical alcoyle, linéaire ou ramifié, renfermant de 1 à 5 atomes de carbone, ainsi que leurs sels d'addition avec les acides minéraux ou organiques.

3) L'un quelconque des dérivés répondant à la formule générale (I), dont les noms suivent:
- la (6RS,trans) 6-amino 7-hydroxy 4,5,6,7-tétrahydroimidazo /5,4,1-j-k/ /1/ benzazépin-2(1H)-one;
- la (6RS,trans) 6-éthylamino-7-hydroxy 4,5,6,7-tétrahydroimidazo/5,4,1-j-k/ /1/ benzazépin-2(1H)-one
- la (6RS,trans) 6-méthylamino 7-hydroxy 4,5,6,7-tétrahydroimidazo/5,4,1-j-k/ /1/ benzazépin-2(1H)-one;
- la (6RS,trans) 6-(1-méthyléthyl)amino 7-hydroxy 4,5,6,7-tétrahydroimidazo/5,4,1-j-k/ /1/ benzazépin-2-(1H)-one, ainsi que leurs sels d'addition avec les acides minéraux ou organiques.

4) Procédé de préparation des dérivés de formule générale (I), telle que définie à la revendication 1, ainsi que de leurs sels d'addition avec les acides minéraux on organiques, caractérisé en ce que l'on soumet le produit de formule (II):

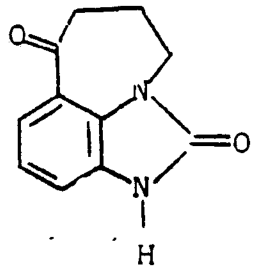

(II)

à l'action d'un nitrite d'alcoyle, de préférence, en présence d'un acide, pour obtenir le produit de formule (III):

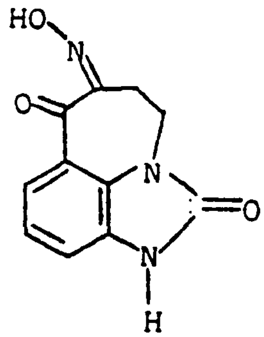

(III)

que l'on réduit, pour obtenir le produit de formule ($I_A$):

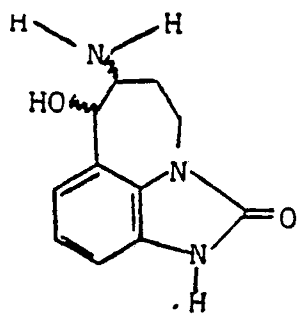

($I_A$)

dans laquelle les traits ondulés ont la signification déjà indiquée, que <u>soit</u> l'on isole et, si désiré, salifie <u>soit</u> l'on soumet ou bien à une réaction d'alcoylation après blocage préalable du groupement $-NH_2$ sous forme de dérivé benzylé, pour obtenir après déblocage, un produit de formule ($I_B$):

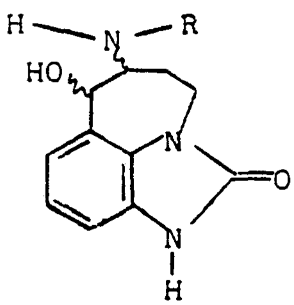

($I_B$)

dans laquelle les traits ondulés ont la signification déjà indiquée et R a la signification indiquée précédemment, à l'exception d'un atome d'hydrogène que l'on isole et, si désiré, salifie, ou bien à l'action d'un dérivé carbonylé de formule (IV):

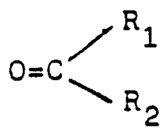

(IV)

dans laquelle $R_1$ et $R_2$ sont tels que 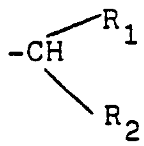

a la signification de R déjà indiquée, à l'exception d'un atome d'hydrogène et d'un radical méthyle, en présence d'une amine tertiaire, puis à l'action d'un agent de réduction, pour obtenir un produit de formule ($I_C$):

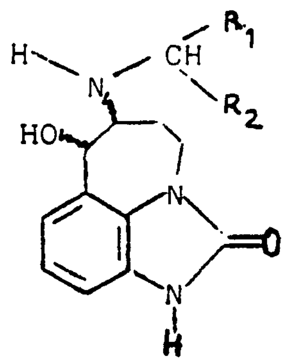

($I_C$)

dans laquelle 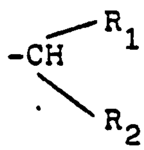

est défini comme ci-dessus, que l'on isole et, si desiré, salifie.

5) Procédé selon la revendication 4, caractérisé en ce que pour préparer le produit de formule (II), l'on fait réagir le produit de formule (V):

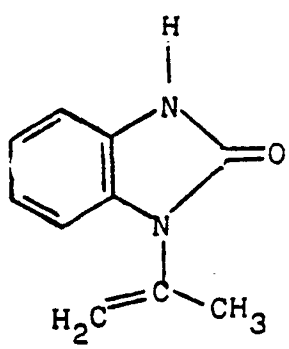

(V)

avec un 4-halogénobutyrate d'alcoyle de formule (VI):

Hal-$(CH_2)_3$-COOalc (VI)

dans laquelle Hal représente un atome de chlore, de brome ou d'iode et alc un radical alcoyle renfermant de 1 à 4 atomes de carbone, puis hydrolyse en milieu acide le produit résultant de formule (VII):

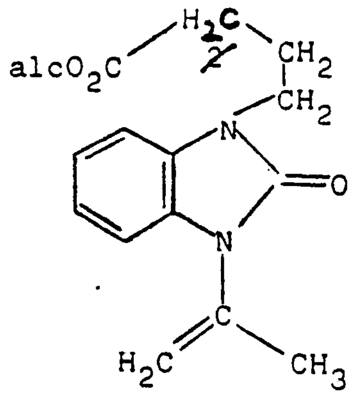

(VII)

dans laquelle alc est défini comme précédemment, pour obtenir le produit de formule (VIII):

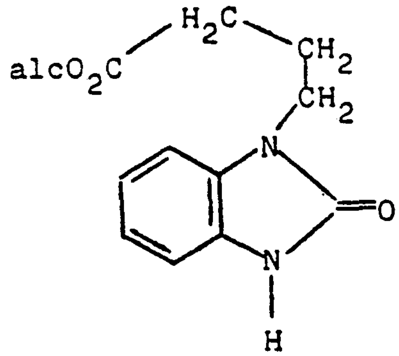

(VIII)

dont on saponifie la fonction ester pour obtenir l'acide de formule (IX):

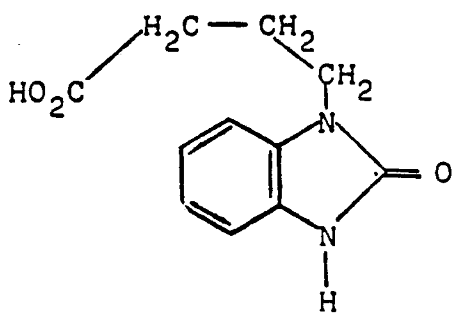

(IX)

dont on cyclise la chaîne latérale pour obtenir le produit de formule II.

6) Médicaments, caractérisés en ce qu'ils sont constitués par les dérivés de la 6-amino 7-hydroxy 4,5,6,7-tétrahydro-imidazo/4,5,1-j-k/ /1/ benzazépin-2(1H)-one, tels que définis par la formule générale (I) de la revendication 1, ainsi que par leurs sels d'addition avec les acides pharmaceutiquement acceptables.

7) Médicaments, caractérisés en ce qu'ils sont constitués par les dérivés de la 6-amino 7-hydroxy 4,5,6,7-tétrahydroimidazo /4,5,1-j-k/ /1/ benzazépin-2(1H)-one, tels que définis à la revendication 2 ainsi que par leurs sels d'addition avec les acides pharmaceutiquement acceptables.

8) Médicaments, caractérisés en ce qu'ils sont constitués par les dérivés de la 6-amino 7-hydroxy 4,5,6,7-tétrahydro-imidazo/4,5,1-j-k/ /1/ benzazépin-2(1H)-one, tels que définis à la revendication 3, ainsi que par leurs sels d'addition avec les acides pharmaceutiquement acceptables.

9) Compositions pharmaceutiques caractérisées en ce qu'elles renferment, à titre de principe actif, l'un au moins des médicaments, tels que définis à l'une des revendications 6, 7 ou 8.

**Revendications** pour l'Etat contractant AT

1) Procédé de préparation de dérivés de la 6-amino 7-hydroxy 4,5,6,7-tétrahydro-imidazo/4,5,1-j-k/ /1/ benzazépin 2(1H)-one ainsi que de leurs sels d'addition avec les acides minéraux ou organiques, répondant à la formule générale (I):

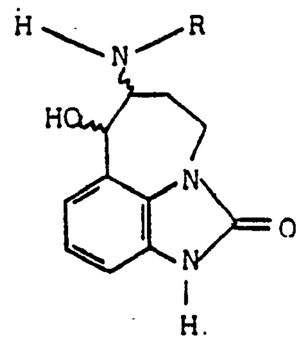

(I)

dans laquelle R représente un atome d'hydrogène, un radical alcoyle, linéaire ou ramifié renfermant de 1 à 8 atomes de carbone, éventuellement substitué par un radical hydroxy, par un radical phényle ou par un radical phényloxy ou un radical cycloalcoyle renfermant de 3 à 7 atomes de carbone, ou un radical 4-pipéridinyle dont l'atome d'azote porte éventuellement un substituant alcoyle renfermant de 1 à 4 atomes de carbone et les traits ondulés signifient que l'hydroxyle en position 7 et le radical amino en position 6 sont de configuration trans, caractérisé en ce que l'on soumet le produit de formule (II):

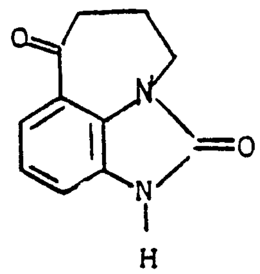

(II)

à l'action d'un nitrite d'alcoyle, de préférence, en présence d'un acide, pour obtenir le produit de formule (III):

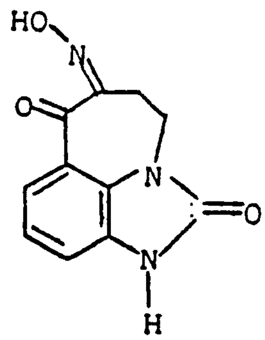

(III)

que l'on réduit, pour obtenir le produit de formule ($I_A$):

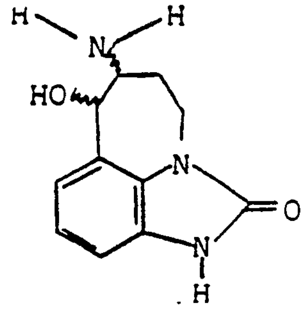

($I_A$)

dans laquelle les traits ondulés ont la signification déjà indiquée, que soit l'on isole et, si désiré, salifie soit l'on soumet ou bien à une réaction d'alcoylation, aprés blocage préalable du groupement $-NH_2$ sous forme de dérivé benzylé, pour obtenir après déblocage, un produit de formule ($I_B$):

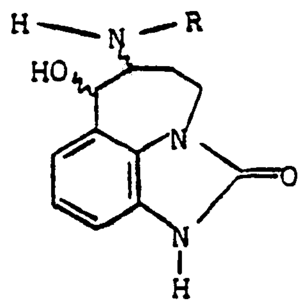

($I_B$)

dans laquelle les traits ondulés ont la signification déjà indiquée et R a la signification indiquée précédemment, à l'exception d'un atome d'hydrogène que l'on isole et, si désiré, salifie, ou bien à l'action d'un dérivé carbonylé de formule (IV):

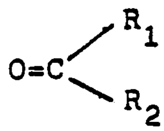

(IV)

dans laquelle $R_1$ et $R_2$ sont tels que 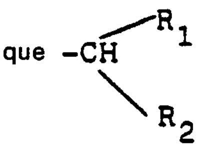

a la signification de R déjà indiquée, à l'exception d'un atome d'hydrogène et d'un radical méthyle, en présence d'une amine tertiaire, puis à l'action d'un agent de réduction, pour obtenir un produit de formule ($I_C$):

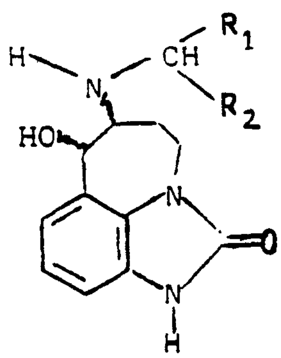

($I_C$)

dans laquelle 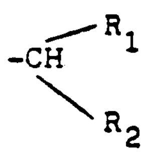

est defini comme ci-dessus, que l'on isole et, si désiré, salifie.

2) Procédé selon la revendication 1 pour la préparation des dérivés répondant à la formule I, dans laquelle R représente un atome d'hydrogène ou un radical alcoyle, linéaire ou ramifié, renfermant de 1 à 5 atomes de carbone ainsi que de leurs sels, caractérisé en ce que l'on soumet le produit de formule (II):

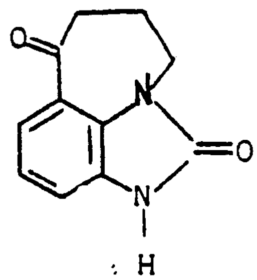

(II)

à l'action d'un nitrite d'alcoyle, de préférence, en présence d'un acide, pour obtenir le produit de formule (III):

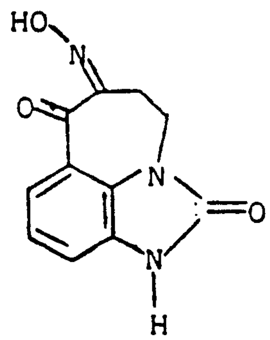

(III)

que l'on réduit, pour obtenir le produit de formule ($I_A$):

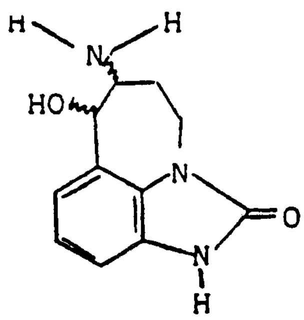

($I_A$) :

dans laquelle les traits ondulés ont la signification déjà indiquée, que soit l'on isole et, si désiré, salifie, soit l'on soumet ou bien à une réaction d'alcoylation après blocage préalable du groupement $-NH_2$ sous forme de dérivé benzyle, pour obtenir après déblocage un produit de formule ($I_B$):

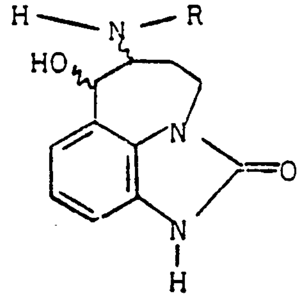

($I_B$)

dans laquelle les traits ondulés ont la signification déjà indiquée, et R a la signification indiquée précédemment, à l'exception d'un atome d'hydrogène, que l'on isole, et si désiré, salifie, ou bien à l'action d'un dérivé carbonylé de formule (IV)

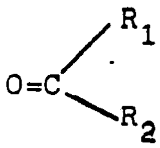

(IV)

dans laquelle $R_1$ et $R_2$ sont tels que 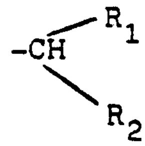

représente un radical alcoyle, lineaire ou ramifié renfermant de 1 à 5 atomes de carbone, en présence d'une amine tertiaire, puis à l'action d'un agent de réduction, pour obtenir un produit de formule ($I_C$):

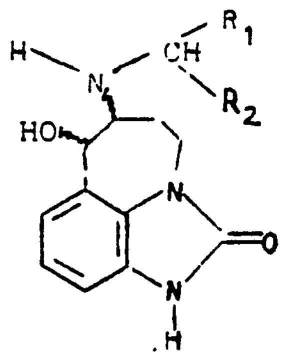

($I_C$)

dans laquelle 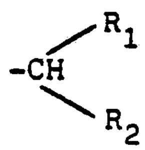

est défini comme ci-dessus, que l'on isole et, si désiré, salifie.

3) Procédé selon la revendication 1 ou 2, caractérisé en ce que:

- la réaction avec le nitrite d'alkyle est effectuée à l'aide de nitrite de tert-butyle en présence d'acide chlorhydrique;

- la réduction du produit de formule (III) est effectuée par hydrogénation catalytique suivie ou non de l'action du borohydrure de sodium;

- la réaction d'alcoylation du produit de formule ($I_A$) est effectuée après blocage sous forme de dérivé N-benzylé, du dérivé de formule ($I_A$), par action d'un halogénure d'alcoyle approprié en présence ou non d'un agent fixateur d'acide, suivie de l'élimination du groupement protecteur;,

- l'amine tertiaire que l'on fait réagir avec le dérivé carbonylé de formule (IV) est la triéthylamine;

- l'agent de réduction que l'on fait réagir sur le produit issu de l'action du produit de formule (IV) avec le produit de formule ($I_A$) est un borohydrure ou cyanoborohydrure alcalin.

4) Procédé selon l'une quelconque des revendications 1 à 3, caractérisé en ce que pour préparer le produit de formule (II) l'on fait réagir le produit de formule (V):

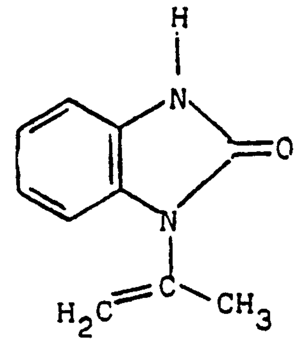

avec un 4-halogénobutyrate d'alcoyle de formule (VI):

$Hal-(CH_2)_3-COOalc$ (VI)

dans laquelle Hal représente un atome de chlore, de brome ou d'iode et alc un radical alcoyle renfermant de 1 à 4 atomes de carbone, puis hydrolyse en milieu acide le produit résultant de formule (VII):

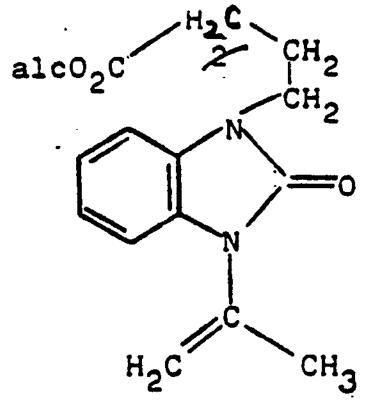

(VII)

dans laquelle alc est défini comme précédemment, pour obtenir le produit de formule (VIII):

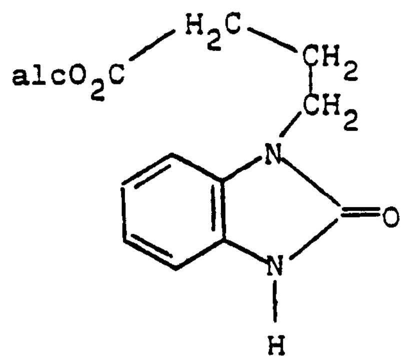

(VIII)

dont on saponifie la fonction ester pour obtenir l'acide de formule (IX):

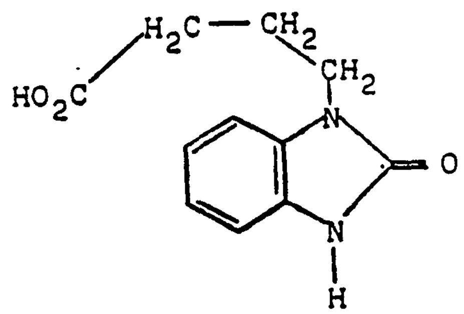

(IX)

dont on cyclise la chaîne latérale pour obtenir le produit de formule II.

5) Procédé selon la revendication 4, caractérisé en ce que

- l'halogénobutyrate d'alcoyle est un bromobutyrate de méthyle ou d'éthyle et la condensation est effectuée en présence d'un hydrure alcalin;
- l'hydrolyse du produit de formule (VII) est effectuée en présence d'acide sulfurique, la réaction étant effectuée au sein d'éthanol;
- la saponification du produit de formule (VIII) est effectuée par la soude ou la potasse au sein de méthanol ou d'éthanol;
- la réaction de cyclisation de la chaîne latérale du produit de formule (IX) est effectuée soit en préparant un chlorure d'acide, que l'on traite par du chlorure d'aluminium, au sein de chlorure de méthylène ou de dichloréthane, soit par action d'acide polyphosphorique.

6) Procédé selon l'une quelconque des revendications 1 à 5, caractérisé en ce que les produits de départ sont choisis de manière telle que l'on prépare l'un quelconque des dérivés répondant à la formule générale (I), dont les noms suivent:

- la (6RS,trans) 6-amino 7-hydroxy 4,5,6,7-tétrahydroimidazo /5,4,1-j-k/ /1/ benzazépin-2(1H)-one;
- la (6RS,trans) 6-éthylamino 7-hydroxy 4,5,6,7-tétrahydroimidazo/5,4,1-j-k/ /1/ benzazépin-2(1H)-one;
- la (6RS,trans) 6-méthylamino 7-hydroxy 4,5,6,7-tétrahydroimidazo/5,4,1-j-k/ /1/ benzazépin-2(1H)-one;
- la (6RS,trans) 6-(1-méthyléthyl)amino 7-hydroxy 4,5,6,7-tétrahydroimidazo/5,4,1-j-k/ /1/ benzazépin-2-(1H)-one,

ainsi que leurs sels d'addition avec les acides minéraux ou organiques.

7) Procédé selon la revendication 2,caractérisé en ce que les produits de départ sont choisis de manière telle que l'on prépare l'un quelconque des dérivés répondant à la formule générale (I), dont les noms suivent:

- la (6RS,trans) 6-amino 7-hydroxy 4,5,6,7-tétrahydroimidazo /5,4,1-j-k/ /1/ benzazépin-2(1H)-one;
- la (6RS,trans) 6-éthylamino 7-hydroxy 4,5,6,7-tétrahydroimidazo/5,4,1-j-k/ /1/ benzazépin-2(1H)-one;
- la (6RS,trans) 6-méthylamino 7-hydroxy 4,5,6,7-tétrahydroimidazo/5,4,1-j-k/ /1/ benzazépin-2(1H)-one;
- la (6RS,trans) 6-(1-méthyléthyl)amino 7-hydroxy 4,5,6,7-tétrahydroimidazo/5,4,1-j-k/ /1/ benzazépin-2-(1H)-one,

ainsi que leurs sels d'addition avec les acides minéraux ou organiques.

**Patentansprüche** für die Vertragsstaaten: BE, CH, DE, FR, GB, IT, LI,

LU, NL, SE

1. 6-Amino-7-hydroxy-4,5,6,7-tetrahydro-imidazo-[4,5,1-j-k]-[1]-benzazepin-2(1H)-on-Derivate sowie deren Additionssalze mit Mineral- oder organischen Säuren, dadurch gekennzeichnet, daß sie der allgemeinen Formel (I)

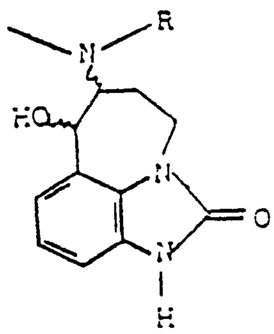

(I)

entsprechen, worin R ein Wasserstoffatom, einen linearen oder verzweigten Alkylrest mit 1 bis 8 Kohlenstoffatomen, der gegebenenfalls durch eine Hydroxygruppe, einen Phenylrest oder durch einen Phenyloxyrest substituiert ist, einen Cycloalkylrest mit 3 bis 7 Kohlenstoffatomen oder einen 4-Piperidinylrest, dessen Stickstoffatom gegebenenfalls einen Alkylsubstituenten mit 1 bis 4 Kohlenstoffatomen enthält, bedeutet und die gewellten Linien anzeigen, daß sich die Hydroxylgruppe in 7-Stellung und die Aminogruppe in 6-Stellung in trans-Konfiguration befinden.

2. Derivate der allgemeinen Formel (I) gemäß Anspruch 1, worin R ein Wasserstoffatom oder einen linearen oder verzweigten Alkylrest mit 1 bis 5 Kohlenstoffatomen bedeutet, sowie deren Additionssalze mit Mineral- oder organischen Säuren.

3. Eihes der Derivate der allgemeinen Formel (I) mit den folgenden Bezeichnungen:
(6RS,trans)-6-Amino-7-hydroxy-4,5,6,7-tetrahydro-imidazo[5,4,1-j-k]-[1]-benzazepin-2(1H)-on;
(6RS,trans)-6-Ethylamino-7-hydroxy-4,5,6,7-tetra-hydro-imidazo[5,4,1-j-k]-[1]-benzazepin-2(1H)-on;
(6RS,trans)-6-Methylamino-7-hydroxy-4,5,6,7-tetrahydro-imidazo[5,4,1-j-k]-[1l]-benzazepin-2(1H)-on;
(6RS,trans)-6-(1-Methylethyl)-amino-7-hydroxy-4,5,6,7-tetrahydro-imidazo[5,4,1-j-k]-[1]-benzazepin-2(1H)-on,
sowie deren Additionssalze mit Mineral- oder organischen Säuren.

4. Verfahren zur Herstellung der Derivate der allgemeinen Formel (I) gemäß Anspruch 1 sowie von deren Additionssalzen mit Mineral- oder organischen Säuren, dadurch gekennzeichnet, daß man das Produkt der Formel (II)

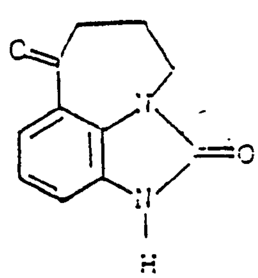

(II)

der Einwirkung eines Alkylnitrits, vorzugsweise in Anwesenheit einer Säure, unterzieht, um das Produkt der Formel (III)

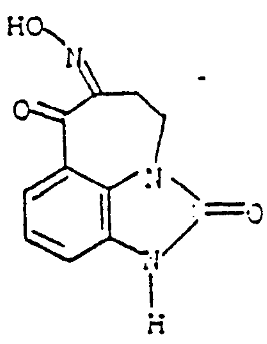

(III)

zu erhalten, das man reduziert, um das Produkt der Formel ($I_A$)

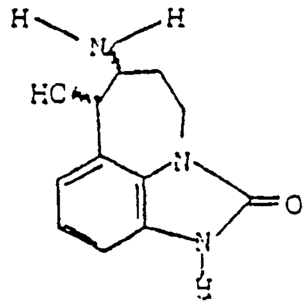

($I_A$)

zu erhalten, worin die gewellten Linien die angegebene Bedeutung besitzen, das man entweder isoliert und gewünschtenfalls in ein Salz überführt, oder entweder einer Alkylierungsreaktion nach vorhergehender Blockierung der -$NH_2$ -Gruppe, in Form des Benzylderivats, unterzieht, um nach der Deblockierung ein Produkt der Formel ($I_B$)

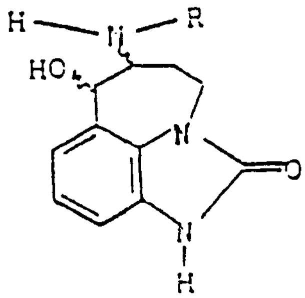

($I_B$)

worin die gewellten Linien die angegebene Bedeutung besitzen und R die vorstehend angegebene Bedeutung mit Ausnahme eines Wasserstoffatoms besitzt, zu erhalten, das man isoliert und gewünschtenfalls in ein Salz überführt oder der Einwirkung eines Carbonylderivats der Formel (IV)

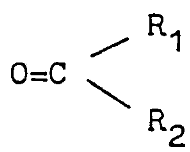

(IV)

worin $R_1$ und $R_2$ derart sind, daß 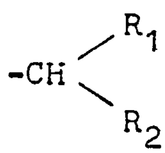 die für R

die für R angegebene Bedeutung mit Ausnahme eines Wasserstoffatoms und eines Methylrestes besitzt, in Anwesenheit eines tertiären Amins und dann der Einwirkung eines Reduktionsmittels unterzieht, um ein Produkt der Formel ($I_C$)

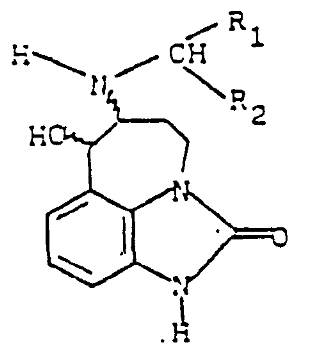

(I$_C$)

zu erhalten, worin

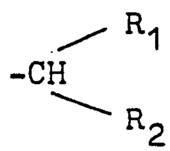

wie vorstehend definiert

ist, das man isoliert und gewünschtenfalls in ein Salz überführt.

5. Verfahren gemäß Anspruch 4, dadurch gekennzeichnet, daß man zur Herstellung des Produkts der Formel (II) das Produkt der Formel (V)

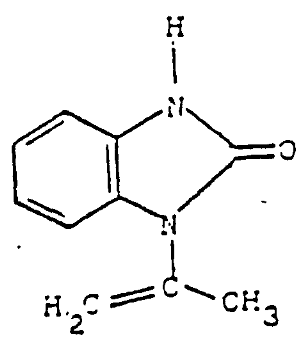

(V)

mit einem Alkyl-4-halogenbutyrat der Formel (VI)

Hal-$(CH_2)_3$-COOalc (VI)

worin Hal ein Chlor-, Brom- oder Jodatom bedeutet und alc einen Alkylrest mit 1 bis 4 Kohlenstoffatomen darstellt, umsetzt, danach das entstandene Produkt der Formel (VII)

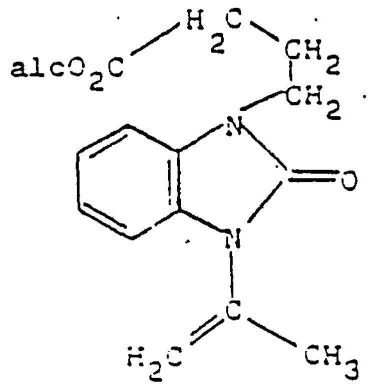

(VII)

worin alc wie vorstehend definiert ist, in saurem Milieu hydrolysiert, um das Produkt der Formel (VIII)

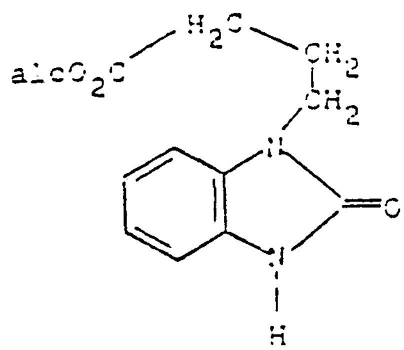

(VIII)

zu erhalten, dessen Esterfunktion man verseift, um die Säure der Formel (IX)

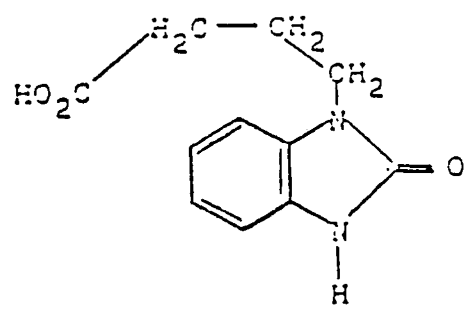

(IX)

zu erhalten, deren Seitenkette man cyclisiert, um das Produkt der Formel (II) zu erhalten.

6. Arzneimittel, dadurch gekennzeichnet, daß sie aus den neuen 6-Amino-7-hydroxy-4,5,6,7-tetrahydro-imidazo[4,5,1-j-k]-[1]-benzazepin-2(1H)-on-Derivaten der allgemeinen Formel (I) gemäß Anspruch 1 sowie aus deren Additionssalzen mit pharmazeutisch verträglichen Säuren bestehen.

7. Arzneimittel, dadurch gekennzeichnet, daß sie aus den 6-Amino-7-hydroxy-4,5,6,7-tetrahydro-imidazo-[4,5,1-j-k]-[1]-benzazepin-2(1H)-on-Derivaten gemäß Anspruch 2 sowie aus deren Additionssalzen mit pharmazeutisch verträglichen Säuren bestehen.

8. Arzneimittel, dadurch gekennzeichnet, daß sie aus den 6-Amino-7-hydroxy-4,5,6,7-tetrahydro-imidazo-[4,5,1-j-k]-[1]-benzazepin-2(1H)-on-Derivaten gemäß Anspruch 3 sowie aus deren Additionssalzen mit pharmazeutisch verträglichen Säuren bestehen.

9. Pharmazeutische Zusammensetzungen, dadurch gekennzeichnet, daß sie als Wirkstoff zumindest eines der Arzneimittel gemäß einem der Ansprüche 6, 7 oder 8 enthalten.

**Patentansprüche** für den Vertragsstaat: AT

1. Verfahren zur Herstellung von 6-Amino-7-hydroxy-4,5,6,7-tetrahydro-imidazo[4,5,1-j-k]-[1]-benzazepin-2(1H)-on-Derivaten sowie von deren Additionssalzen mit Mineral- oder organischen Säuren der allgemeinen

Formel (I)

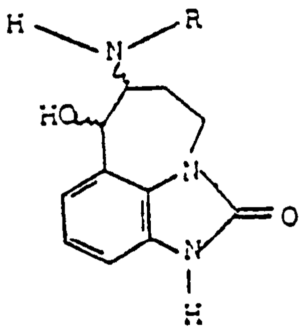

(I)

worin R ein Wasserstoffatom, einen linearen oder verzweigten Alkylrest mit 1 bis 8 Kohlenstoffatomen, der gegebenenfalls durch eine Hydroxygruppe, einen Phenylrest oder durch einen Phenyloxyrest substituiert ist, einen Cycloalkylrest mit 3 bis 7 Kohlenstoffatomen oder einen 4-Piperidinylrest, dessen Stickstoffatom gegebenenfalls einen Alkylsubstituenten mit 1 bis 4 Kohlenstoffatomen enthält, bedeutet und die gewellten Linien anzeigen, daß sich die Hydroxylgruppe in 7-Stellung und die Aminogruppe in 6-Stellung in trans-Konfiguration befinden, dadurch gekennzeichnet, daß man das Produkt der Formel (II)

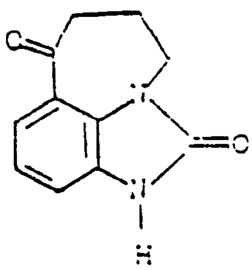

(II)

der Einwirkung eines Alkylnitrits, vorzugsweise in Anwesenheit einer Säure, unterzieht, um das Produkt der Formel (III)

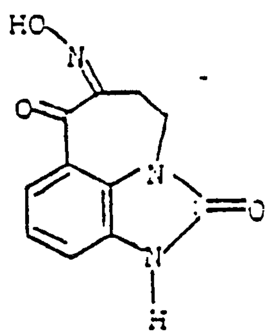

(III)

zu erhalten, das man reduziert, um das Produkt der Formel ($I_A$)

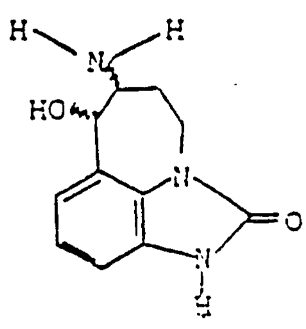

($I_A$)

zu erhalten, worin die gewellten Linien die angegebene Bedeutung besitzen, das man entweder isoliert und gewünschtenfalls in ein Salz überführt, oder entweder einer Alkylierungsreaktion nach vorhergehender Blockierung der $-NH_2$-Gruppe, in Form des Benzylderivats, unterzieht, um nach der Deblockierung ein Produkt

der Formel ($I_B$)

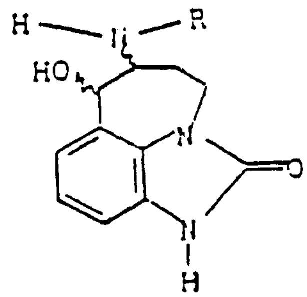

($I_B$)

worin die gewellten Linien die angegebene Bedeutung besitzen und R die vorstehend angegebene Bedeutung mit Ausnahme eines Wasserstoffatoms besitzt, zu erhalten, das man isoliert und gewünschtenfalls in ein Salz überführt oder der Einwirkung eines Carbonylderivats der Formel (IV)

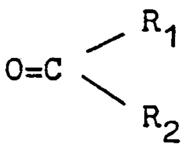

(IV)

worin $R_1$ und $R_2$ derart sind, daß 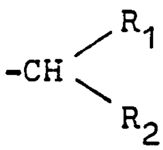

die für R angegebene Bedeutung mit Ausnahme eines Wasserstoffatoms und eines Methylrestes besitzt, in Anwesenheit eines tertiären Amins und dann der Einwirkung eines Reduktionsmittels unterzieht, um ein Produkt der Formel ($I_C$)

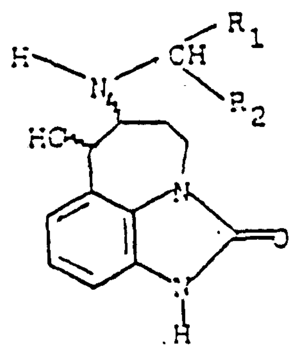

($I_C$)

zu erhalten, worin 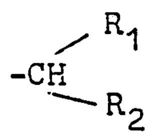

wie vorstehend definiert ist, das man isoliert und gewünschtenfalls in ein Salz überführt.

2. Verfahren gemäß Anspruch 1 zur HerstellLung der Derivate der Formel I, worin R ein Wasserstoffatom oder einen linearen oder verzweigten Alkylrest mit 1 bis 5 Kohlenstoffatomen bedeutet, sowie von deren Salzen, dadurch gekennzeichnet, daß man das Produkt der Formel (II)

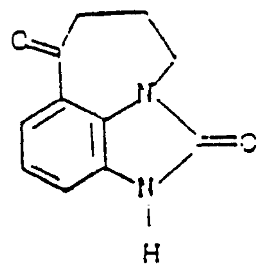

(II)

der Einwirkung eines Alkylnitrits, vorzugsweise in Anwesenheit einer Säure, unterzieht, um das Produkt der Formel (III)

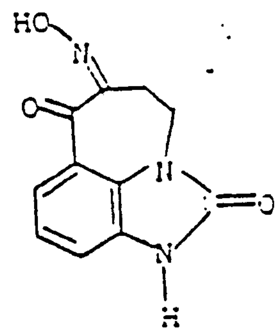

(III)

zu erhalten, das man reduziert, um das Produkt der Formel ($I_A$)

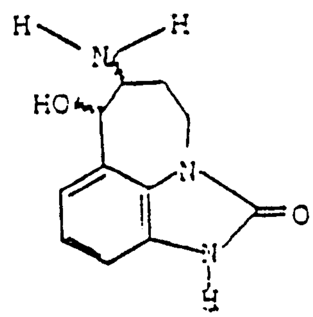

($I_A$)

zu erhalten, worin die gewellten Linien die angegebene Bedeutung besitzen, das man entweder isoliert und gewünschtenfalls in ein Salz überführt, oder entweder einer Alkylierungsreaktion nach vorhergehender Blockierung der $-NH_2$ -Gruppe, in Form des Benzylderivats, unterzieht, um nach der Deblockierung ein Produkt der Formel ($I_B$)

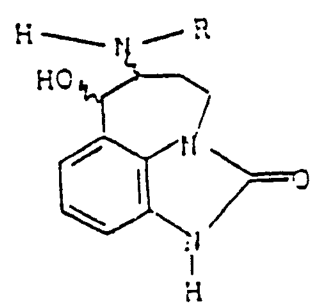

($I_B$)

worin die gewellten Linien die angegebene Bedeutung besitzen und R die vorstehend angegebene Bedeutung mit Ausnahme eines Wasserstoffatoms besitzt, zu erhalten, das man isoliert und gewünschtenfalls in ein Salz überführt oder der Einwirkung eines Carbonylderivats der Formel (IV)

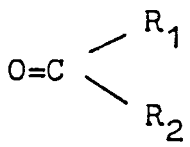

(IV)

worin $R_1$ und $R_2$ derart sind, daß 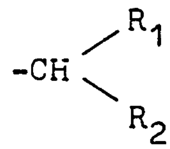

einen linearen oder verzweigten Alkylrest mit 1 bis 5 Kohlenstoffatomen darstellt, in Anwesenheit eines tertiären Amins und danach der Einwirkung eines Reduktionsmittels unterzieht, um ein Produkt der Formel ($I_C$)

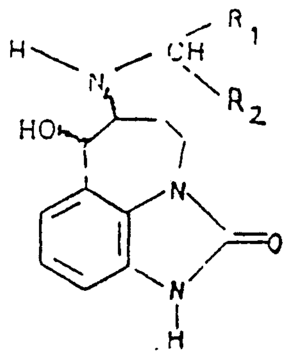

($I_C$)

zu erhalten, worin 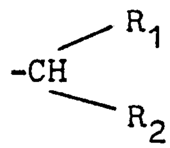

wie vorstehend definiert ist, das man isoliert und gewünschtenfalls in ein Salz überführt.

3. Verfahren gemäß Anspruch 1 oder 2, dadurch gekennzeichnet, daß

die Umsetzung mit dem Alkylnitrit mit Hilfe von tert.-Butylnitrit in Anwesenheit von Chlorwasserstoffsäure durchgeführt wird,

die Reduktion des Produkts der Formel (III) durch katalytische Hydrierung bewirkt wird, woran sich gegebenenfalls die Einwirkung von Natriumborhydrid anschließt,

die Alkylierungsreaktion des Produkts der Formel ($I_A$) nach Blockierung in Form des N-Benzylderivats des Derivats der Formel ($I_A$) durch Umsetzung mit einem geeigneten Alkylhalogenid in Anwesenheit oder Abwesenheit eines säurebindenden Mittels durchgeführt wird, woran sich die Eliminierung der Schutzgruppe anschließt,

das tertiäre Amin, das man mit dem Carbonylderivat der Formel (IV) umsetzt, Triethylamin ist,

das Reduktionsmittel, das man mit dem der Umsetzung des Produkts der Formel (IV) mit dem Produkt der Formel ($I_A$) entstammenden Produkt umsetzt, ein Alkaliborhydrid oder Alkalicyanoborhydrid ist.

4. Verfahren gemäß einem der Ansprüche 1 bis 3, dadurch gekennzeichnet, daß man zur Herstellung des Produkts der Formel (II) das Produkt der Formel (V)

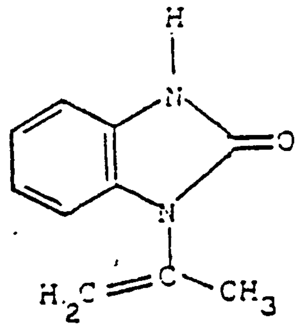

(V)

mit einem Alkyl-4-halogenbutyrat der Formel (VI)

Hal-$(CH_2)_3$-COOalc (VI)

worin Hal ein Chlor-, Brom- oder Jodatom bedeutet und alc einen Alkylrest mit 1 bis 4 Kohlenstoffatomen darstellt, umsetzt, danach das entstandene Produkt der Formel (VII)

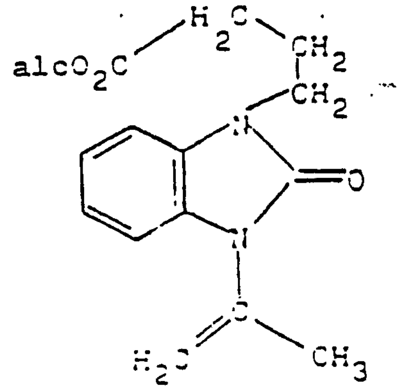

(VII)

worin alc wie vorstehend definiert ist, in saurem Milieu hydrolysiert, um das Produkt der Formel (VIII)

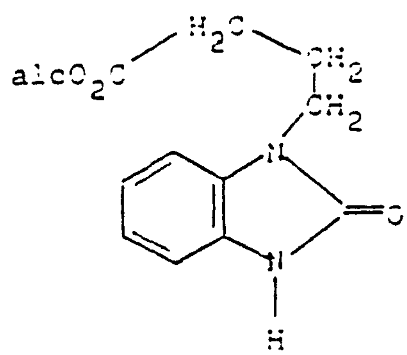

(VIII)

zu erhalten, dessen Esterfunktion man verseift, um die Säure der Formel (IX)

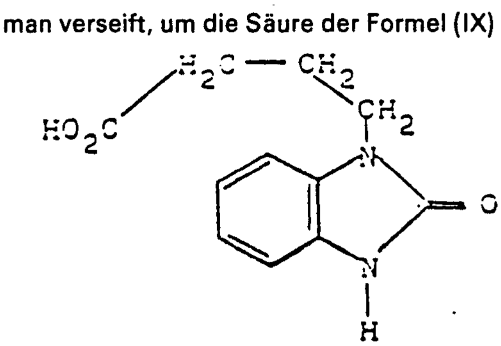

(IX)

zu erhalten, deren Seitenkette man cyclisiert, um das Produkt der Formel (II) zu erhalten.

5. Verfahren gemäß Anspruch 4, dadurch gekennzeichnet, daß

das Aklylhalogenobutyrat ein Methyl- oder Ethylbromobutyrat ist und die Kondensation in Anwesenheit eines Alkalihydrids durchgeführt wird,

die Hydrolyse des Produkts der Formel (VII) in Anwesenheit von Schwefelsäure durchgeführt wird, wobei

die Reaktion in einem Ethanol-Medium erfolgt,
die Verseifung des Produkts der Formel (VIII) mit Natron- oder Kalilauge in einem Methanol- oder Ethanol-Medium durchgeführt wird,
die Cyclisierungsreaktion der Seitenkette des Produkts der Formel (IX) entweder durchgeführt wird, indem man ein Säurechlorid herstellt, das man mit Aluminiumchlorid in einem Methylenchlorid- oder Dichlorethan-Medium behandelt, oder durch Umsetzung mit Polyphosphorsäure.

6. Verfahren gemäß einem der Ansprüche 1 bis 5, dadurch gekennzeichnet, daß die Ausgangsprodukte derart ausgewählt werden, daß man eines der Derivate der allgemeinen Formel (I) mit den folgenden Bezeichnungen
(6RS,trans)-6-Amino-7-hydroxy-4,5,6,7-tetrahydro-imidazo[5,4,1-j-k]-[1]-benzazepin-2(1H)-on;
(6RS,trans)-6-Ethylamino-7-hydroxy-4,5,6,7-tetra-hydro-imidazo[5,4,1-j-k]-[1]-benzazepin-2(1H)-on;
(6RS,trans)-6-Methylamino-7-hydroxy-4,5,6,7-tetra-hydro-imidazo[5,4,1-j-k]-[1]-benzazepin-2(1H)-on;
(6RS,trans)-6-(1-Methylethyl)-amino-7-hydroxy-4,5,6,7-tetrahydro-imidazo[5,4,1-j-k]-[1]-benzazepin-2-(1H)-on,
sowie deren Additionssalze mit Mineral- oder organischen Säuren herstellt.

7. Verfahren gemäß Anspruch 2, dadurch gekennzeichnet, daß die Ausgangsprodukte derart ausgewählt werden, daß man eines der Derivate der allgemeinen Formel (I) mit den folgenden Bezeichnungen
(6R8,trans)-6-Amino-7-hydroxy-4,5,6,7-tetrahydro-imidazo[5,4,1-j-k]-[1]-benzazepin-2(1H)-on;
(6RS,trans)-6-Ethylamino-7-hydroxy-4,5,6,7-tetra-hydro-imidazo[5,4,1-j-k]-[1]-benzazepin-2(1H)-on;
(6RS,trans)-6-Methylamino-7-hydroxy-4,5,6,7-tetrahydro-imidazo[5,4,1-j-k]-[1]-benzazepin-2(1H)-on;
(6RS,trans)-6-(1-Methylethyl)-amino-7-hydroxy-4,5,6,7-tetrahydro-imidazo[5,4,1-j-k]-[1]-benzazepin-2(1H)-on
sowie deren Additionssalze mit Mineral- oder organischen Säuren herstellt.

**Claims** for the contracting states BE CH DE FR GB IT LI LU NL SE

1) The derivatives of 6-amino-7-hydroxy-4,5,6,7-tetrahydroimidazo [4,5,1-j-k]-[1]-benzazepin-2-(1H)-one as well as their salts of addition with mineral or organic acids, characterized in that they correspond to the general formula (I):

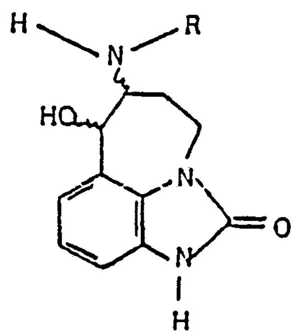

(I)

in which R represents a hydrogen atom, a linear or branched alkyl radical containing from 1 to 8 carbon atoms, possibly substituted by a hydroxy radical, by a phenyl radical or by a phenyloxy radical or a cycloalkyl radical containing from 3 to 7 carbon atoms, or a 4-piperidinyl radical of which the nitrogen atom possibly carries an alkyl substituent containing from 1 to 4 carbon atoms and the wavy lines signify that the hydroxyl in position 7 and the amino radical in position 6 are of trans configuration.

2) The derivatives corresponding to the general formula (I) of claim 1, in which R represents a hydrogen atom or a linear or branched alkyl radical containing from 1 to 5 carbon atoms, as well as their salts of addition with mineral or organic acids.

3) Any one of the derivatives corresponding to the general formula (I) of which the names follow:
- (6RS,trans)-6-amino-7-hydroxy-4,5,6,7-tetrahydroimidazo[5,4,1-j-k]-[1]-benzazepin-2(1H)-one;
- (6RS,trans)-6-ethylamino-7-hydroxy-4,5,6,7-tetrahydroimidazo[5,4,1-j-k]-[1]-benzazepin-2(1H)-one;
- (6RS,trans)-6-methylamino-7-hydroxy-4,5,6,7-tetrahydroimidazo[5,4,1-j-k]-[1]-benzazepin-2(1H)-one;
- (6RS,trans)-6-(1-methylethyl)amino-7-hydroxy-4,5,6,7-tetrahydroimidazo[5,4,1-j-k]-[1]-benzazepin-2(1H)-one,

as well as their salts of addition with mineral or organic acids.

4) Preparation process for the derivatives with the general formula (I), as defined in claim 1, as well as their salts of addition with mineral or organic acids, characterized in that the product with the formula (II):

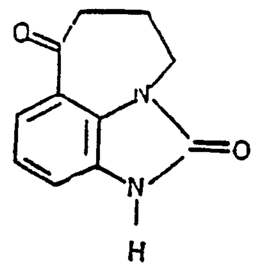

(II)

is submitted to the action of an alkyl nitrite, preferably in the presence of an acid, so as to obtain the product with the formula (III):

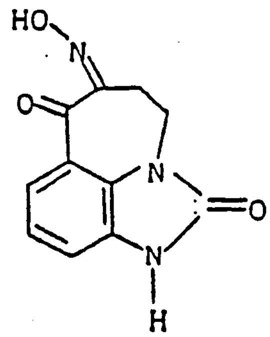

(III)

which is reduced, so as to obtain the product with the formula ($I_A$):

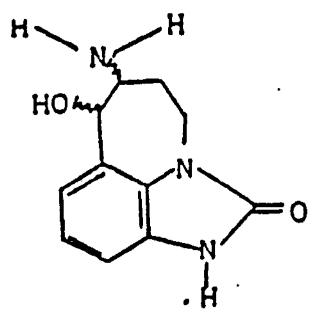

($I_A$)

in which the wavy lines have the significance already indicated, which <u>either</u> is isolated and, if desired, salified, <u>or</u> is submitted either to an alkylation reaction, after prior blockage of the $-NH_2$ group in the form of a benzyl derivative, so as to obtain, after removal of the blockage, a product with the formula ($I_B$):

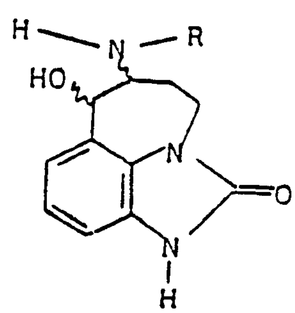

($I_B$)

in which the wavy lines have the significance already indicated and R has the significance indicated previously, with the exception of a hydrogen atom, which is isolated and, if desired, salified, or to the action of a carbonyl derivative with the formula (IV):

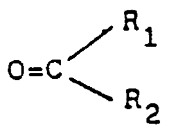

(IV)

in which $R_1$ and $R_2$ are such that 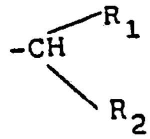

has the significance already indicated for R, with the exception of a hydrogen atom and of a methyl radical, in the presence of a tertiary amine, then to the action of a reducing agent, so as to obtain a product with the formula ($I_C$):

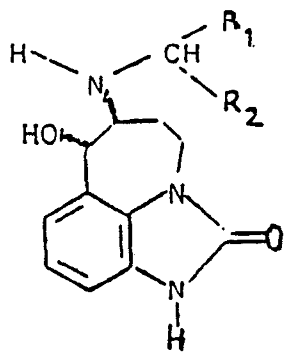

($I_C$)

in which 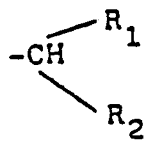

is defined as above, which is isolated and, if desired, salified.

5) Process according to claim 4, characterized in that to prepare the product with the formula (II), the product with the formula (V):

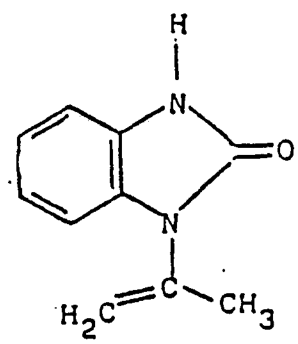

(V)

is made to react with an alkyl-4-halogenobutyrate with the formula (VI)

$Hal-(CH_2)_3-COOalk$ (VI)

in which Hal represents a chlorine, bromine or iodine atom and alk represents an alkyl radical containing from 1 to 4 carbon atoms, then the resulting product with the formula (VII):

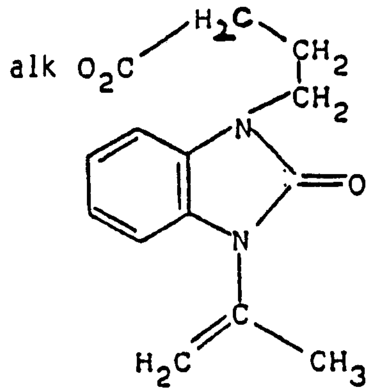

(VII)

in which alk is defined as previously, is hydrolyzed in an acid medium, so as to obtain the product with the formula (VIII):

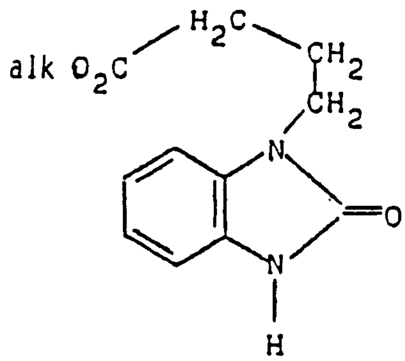

(VIII)

of which the ester function is saponified so as to obtain the acid with the formula (IX):

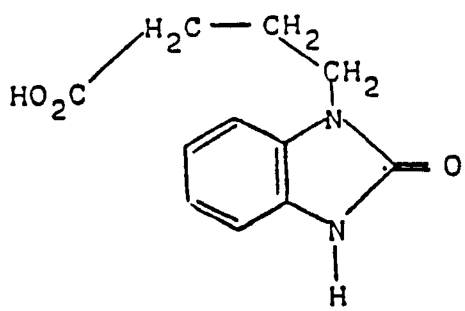

(IX)

of which the lateral chain is cyclized so as to obtain the product with the formula (II).

6) Medicaments, characterized in that they are constituted by the derivatives of 6-amino-7-hydroxy-4,5,6,7-tetrahydroimidazo[4,5,1-j-k]-[1]-benzazepin-2(1H)-one, as defined by the general formula (I) of claim 1, as well as by their salts of addition with pharmaceutically acceptable acids.

7) Medicaments, characterized in that they are constituted by the derivatives of 6-amino-7-hydroxy-4,5,6,7-tetrahydroimidazo[4,5,1-j-k]-[1]-benzazepin-2(1H)-one, as defined in claim 2, as well as by their salts of addition with pharmaceutically acceptable acids.

8) Medicaments, characterized in that they are constituted by the derivatives of 6-amino-7-hydroxy-4,5,6,7-tetrahydroimidazo[4,5,1-j-k]-[1]-benzazepin-2(1H)-one, as defined in claim 3, as well as by their salts of addition with pharmaceutically acceptable acids.

9) Pharmaceutical compositions characterized in that they contain, as active principle, one at least of the medicaments as defined in one of the claims 6, 7 or 8.

**Claims** for the contracting state AT

1) Preparation process for the derivatives of 6-amino-7-hydroxy 4,5,6,7-tetrahydroimidazo[4,5,1-j-k]-[1]-benzazepin-2(1H)-one, as well as their salts of addition with mineral or organic acids, corresponding to the general formula (I):

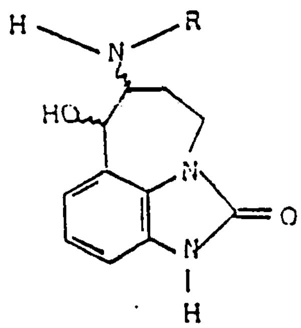

(I)

in which R represents a hydrogen atom, a linear or branched alkyl radical containing from 1 to 8 carbon atoms, possibly substituted by a hydroxy radical, by a phenyl radical or by a phenyloxy radical or a cycloalkyl radical containing from 3 to 7 carbon atoms, or a 4-piperidinyl radical of which the nitrogen atom possibly carries an alkyl substituent containing from 1 to 4 carbon atoms and the wavy lines signify that the hydroxyl in position 7 and the amino radical in position 6 are of trans configuration, characterized in that the product with the formula (II):

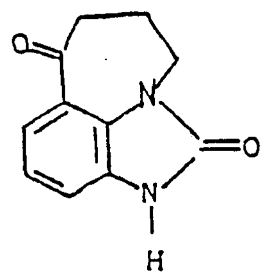

(II)

is submitted to the action of an alkyl nitrite, preferably in the presence of an acid, so as to obtain the product with the formula (III):

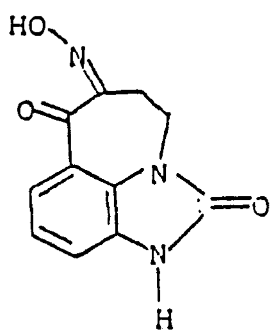

(III)

which is reduced, so as to obtain the product with the formula ($I_A$):

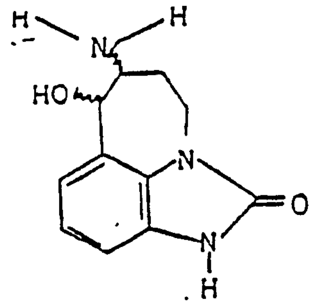

($I_A$) .

in which the wavy lines have the significance already indicated, which <u>either</u> is isolated and, if desired, salified, <u>or</u> is submitted either to an alkylation reaction, after prior blockage of the $-NH_2$ group in the form of a benzyl derivative, so as to obtain, after removal of the blockage, a product with the formula ($I_B$):

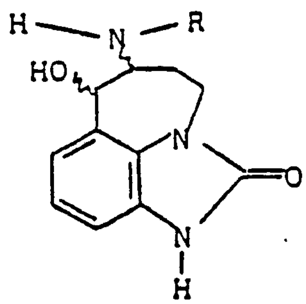

($I_B$)

in which the wavy lines have the significance already indicated and R has the significance indicated previously, with the exception of a hydrogen atom which is isolated and, if desired, salified, or to the action of a carbonyl derivative with the formula (IV):

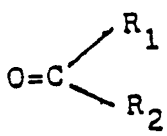

(IV)

in which $R_1$ and $R_2$ are such that 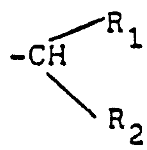

has the significance for R already indicated, with the exception of a hydrogen atom and a methyl radical, in the presence of a tertiary amine, then to to the action of a reducing agent, so as to obtain a product with the formula ($I_C$):

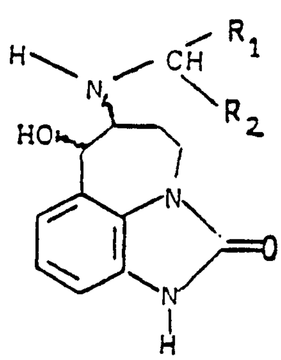

($I_C$)

in which 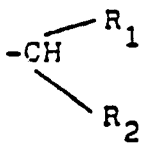

is defined as above which is isolated and, if desired, salified.

2) Process according to claim 1, for the preparation of derivatives corresponding to the formula (I), in which R represents a hydrogen atom or a linear or branched alkyl radical containing from 1 to 5 carbon atoms as well as their salts, characterized in that the product with the formula (II):

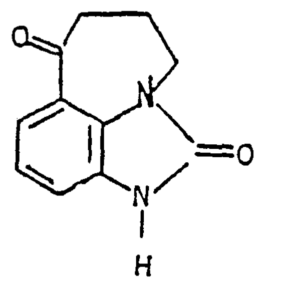

(II)

is submitted to the action of an alkyl nitrite, preferably in the presence of an acid, so as to obtain the product with the formula (III):

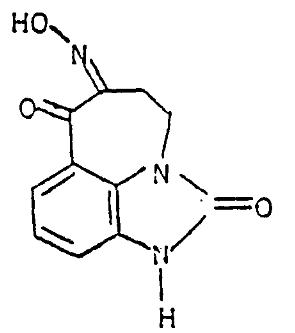

(III)

which is reduced, so as to obtain the product with the formula ($I_A$):

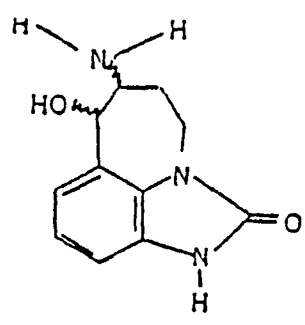

($I_A$)

in which the wavy lines have the significance already indicated, which either is isolated and, if desired, salified, or is submitted either to an alkylation reaction, after prior blockage of the $-NH_2$ group in the form of a benzyl derivative, so as to obtain, after removal of the blockage, a product with the formula ($I_B$):

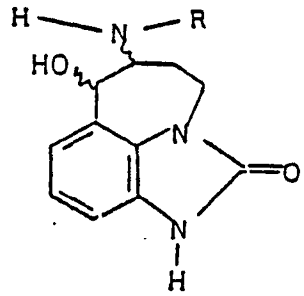

($I_B$)

in which the wavy lines have the significance already indicated and R has the significance indicated previously, with the exception of a hydrogen atom, which is isolated and, if desired, salified, or to the action of a carbonyl derivative with the formula (IV):

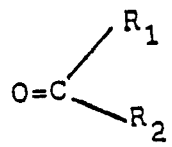

(IV)

in which $R_1$ and $R_2$ are such that 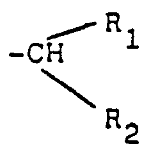

represents a linear or branched alkyl radical containing from 1 to 5 carbon atoms, in the presence of a tertiary amine, then to the action of a reducing agent, so as to obtain a product with the formula ($I_C$):

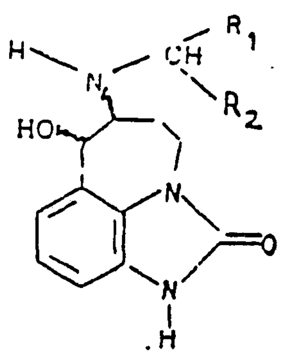

($I_C$)

in which 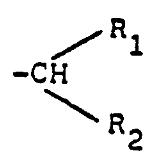

is defined as above, which is isolated and, if desired, salified.

3) Process according to claim 1 or 2, characterized in that:

- the reaction with the alkyl nitrite is carried out by tert-butyl nitrite in the presence of hydrochloric acid;
- the reduction of the product with the formula (III) is carried out by catalytic hydrogenation followed or not with the action of sodium borohydride;
- the alkylation reaction of the product with the formula ($I_A$) is carried out, after blockage of the derivative with the formula ($I_A$), in the form of an N-benzyl derivative, by the action of a suitable alkyl halogenide in the presence or not of an acid fixing agent, followed by the elimination of the protector group;
- the tertiary amine which is made to react with the carbonyl derivative with the formula (IV) is triethylamine;
- the reduction agent which is made to react on the product resulting from the action of the product with the formula (IV) with the product with the formula ($I_A$) is an alkaline borohydride or cyanoborohydride.

4) Process according to any one of the claims 1 to 3, characterized in that to prepare the product with the formula (II), the product with the formula (V):

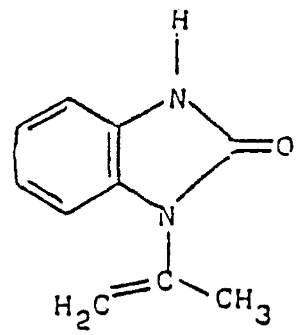

(V)

is made to react with an alkyl-4-halogenobutyrate with the formula (VI)

Hal-$(CH_2)_3$-COOalk (VI)

in which Hal represents a chlorine, bromine or iodine atom and alk represents an alkyl radical containing from 1 to 4 carbon atoms, then the resulting product with the formula (VII):

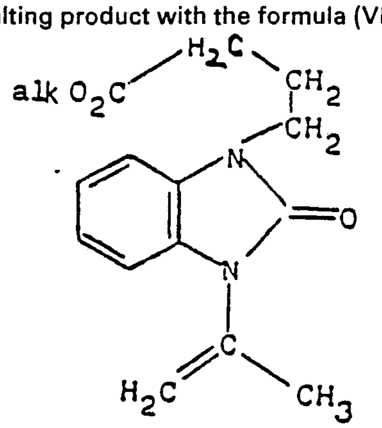

(VII)

in which alk is defined as previously, is hydrolyzed in an acid medium, so as to obtain the product with the formula (VIII):

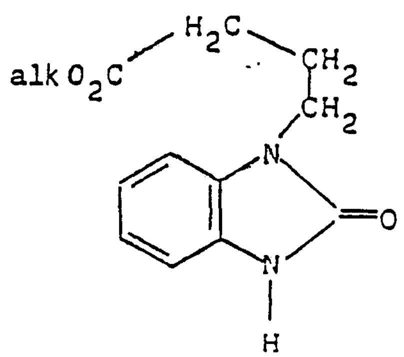

(VIII)

of which the ester function is saponified so as to obtain the acid with the formula (IX):

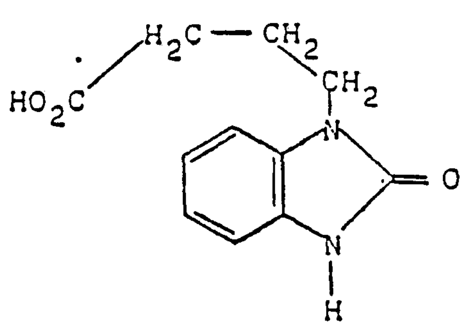

(IX)

of which the lateral chain is cyclized so as to obtain the product with the formula (II).

5) Process according to claim 4, characterized in that:

- the alkyl halogenobutyrate is a methyl or ethyl bromobutyrate and the condensation is carried out in the presence of an alkaline hydride;

- the hydrolysis of the product with the formula (VII) is carried out in the presence of sulphuric acid, the reaction being carried out in ethanol;
- the saponification of the product with the formula (VIII) is carried out by sodium hydroxide or potassium hydroxide in methanol or ethanol;
- the reaction of cyclization of the lateral chain of the product with the formula (IX) is carried out either by preparing an acid chloride, which is treated by aluminium chloride, in methylene chloride or dichloroethane, or by the action of polyphosphoric acid.

6) Process according to any one of the claims 1 to 5, characterized in that the starting products are chosen in such a way that any one of the derivatives corresponding to the general formula (I) are prepared, of which the names follow:
- (6RS,trans)-6-amino-7-hydroxy-4,5,6,7-tetrahydroimidazo[5,4,1-j-k]-[1]-benzazepin-2(1H)-one;
- (6RS,trans)-6-ethylamino-7-hydroxy-4,5,6,7-tetrahydroimidazo[5,4,1-j-k]-[1]-benzazepin-2(1H)-one;
- (6RS,trans)-6-methylamino-7-hydroxy-4,5,6,7-tetrahydroimidazo[5,4,1-j-k]-[1]-benzazepin-2(1H)-one;
- (6RS,trans)-6-(1-methylethyl)amino-7-hydroxy-4,5,6,7-tetrahydroimidazo[5,4,1-j-k]-[1]-benzazepin-2(1H)-one,

as well as their salts of addition with mineral or organic acids..

7) Process according to claim 2, characterized in that the starting products are chosen in such a way that any one of the derivatives corresponding to the general formula (I) are prepared, of which the names follow:
- (6RS,trans)-6-amino-7-hydroxy-4,5,6,7-tetrahydroimidazo[5,4,1-j-k]-[1]-benzazepin-2(1H)-one;
- (6RS,trans)-6-ethylamino-7-hydroxy-4,5,6,7-tetrahydroimidazo[5,4,1-j-k]-[1]-benzazepin-2(1H)-one;
- (6RS,trans)-6-methylamino-7-hydroxy-4,5,6,7-tetrahydroimidazo[5,4,1-j-k]-[1]-benzazepin-2(1H)-one;
- (6RS,trans)-6-(1-methylethyl)amino-7-hydroxy-4,5,6,7-tetrahydroimidazo[5,4,1-j-k]-[1]-benzazepin-2(1H)-one,

as well as their salts of addition with mineral or organic acids.